# EUROPEAN PATENT APPLICATION

(11) **EP 3 617 316 A1**
(43) Date of publication of application: **04.03.2020**
(21) Application number: 18791384.3
(22) Date of filing: 26.04.2018
(51) Int. Cl.: C12N 15/12, A61K 38/36, A61K 39/395, A61K 47/68, A61P 7/04, C07K 14/745, C07K 16/36, C12N 15/13, C12P 21/02

(54) **COAGULATION FACTOR IX WITH IMPROVED PHARMACOKINETICS**

(30) Priority: 27.04.2017 JP 2017088670
(71) Applicant: Chugai Seiyaku Kabushiki Kaisha, Kita-ku Tokyo 115-8543 (JP)
(72) Inventor: IGAWA, Tomoyuki, Gotemba-shi Shizuoka 412-8513 (JP); FUNAKI, Miho, Gotemba-shi Shizuoka 412-8513 (JP); MIYASHITA, Hiroyuki, Gotemba-shi Shizuoka 412-8513 (JP)
(74) Representative: Vossius & Partner Patentanwälte Rechtsanwälte mbB
(86) International application number: PCT/JP2018/016932
(87) International publication number: WO 2018/199214

(57) **Abstract**

The present invention provides a method for improving or controlling the plasma half-life and/or bio-availability of blood coagulation factor IX (FIX), the method comprising modifying the GLA domain. Examples of such modifications include: (i) non-covalent bonding of a GLA-domain-recognizing antibody or an antibody fragment thereof to the GLA domain; (ii) reduced number of Gla residues in the GLA domain, in comparison to that of a native FIX; (iii) either or both of deletion of one or more glutamic acid residues in the GLA domain and substitution of one or more glutamic acid residues in the GLA domain with another amino acid; and (iv) deletion of a part or all of the GLA domain. The present invention also provides a FIX with improved pharmacokinetics which carries such modifications, a pharmaceutical composition containing the FIX as an active ingredient, a method for producing the FIX, and such.

## Description

### Technical Field

The present invention relates to blood coagulation factor IX (FIX) with improved pharmacokinetics, methods for improving the pharmacokinetics of FIX, methods for controlling the pharmacokinetics of FIX, methods for producing FIX with improved pharmacokinetics, complexes formed between FIX and an antibody, pharmaceutical compositions comprising the complex or FIX with improved pharmacokinetics as an active ingredient, and such.

### Background Art

Hemophilia B is a bleeding abnormality caused by congenital defect or decrease in FIX function. Human-derived FIX formulations are ordinarily administered against bleeding in hemophilia B patients (on-demand administration). Furthermore, to prevent bleeding events, FIX formulations are preventively administered intravenously (Non-patent Document 1) (preventive administration). Recombinant FIX formulations have plasma half-life of approximately 17 to 19 hours; therefore, for continuous prevention, FIX formulations are administered to patients two to three times a week (Non-patent Document 1).

Since frequent intravenous injections impose a large burden on the patients, recently, FIX formulations with extended half-life are being developed (Non-patent Document 1). The recently approved FIX-Fc (common name: eftrenonacog alfa; product name: Alprolix) has its half-life extended to approximately 82 hours by linking an Fc of an IgG1 antibody to the C terminus of a single molecule of FIX, and has succeeded in extending the dosage interval to basically once a week. However, since its route of administration is intravenous injection, further reduction of burden on the patients is desired (Non-patent Document 1). FIX-Fc has fused Fc from human IgG, and this extends its half-life by promoting its recycling *via* FcRn following uptake into cells, similarly to human IgG (Non-patent Document 2). Human IgG is taken up by almost any of the distributed cells, such as vascular endothelial cells, but avoids degradation by lysosomes by binding to FcRn under acidic pH in the endosome, being recycled to the cell surface, and dissociating from FcRn under neutral conditions. Because of this recycling action by Fc, the half-life of a human IgG is two to four weeks and is very long (Non-patent Document 3).

In contrast, the half-life of FIX-Fc carrying the same Fc is approximately 82 hours and is very short compared to that of human IgG; however, the reason for this has not been reported so far and is unknown. In addition to FIX-Fc, FIX formulations with extended half-life such as PEGylated FIX and FIX-Albumin have been developed; however, their routes of administration are all intravenous injection, and their half-lives are approximately 93 hours and approximately 92 hours, respectively, and are very short compared to that of human IgG (Non-patent Document 3). The half-life of Certolizumab pegol (product name: Cimzia) which is a PEGylated Fab molecule is approximately 14 days (Non-patent Document 4), and the half-life of albumin is also approximately 19 days (Non-patent Document 5). Compared to those molecules, PEGylated FIX and FIX-Albumin have very short half-lives, but similarly to FIX-Fc, the reason for this has not been reported so far and is unknown.

In this manner, the cause for the failure in achieving sufficiently long half-life even upon fusing with FIX a half-life elongation element such as Fc, PEG, or Albumin, may reside in FIX itself, but the cause has not been elucidated. Likewise, the reason why FIX is not developed as FIX formulations that can be administered subcutaneously may be due to its low bio-availability, but similarly the cause therefor is unknown.

### Citation List

### [Non-Patent Documents]

Non-Patent Document 1: J Blood Med. 2016 Apr 1;7:27-38.
Non-Patent Document 2: Blood. 2010 Mar 11;115(10):2057-64.
Non-Patent Document 3: J Pharm Sci. 2004 Nov;93(11):2645-68.
Non-Patent Document 4: Expert Opin Drug Metab Toxicol. 2015 Feb;11(2):317-27
Non-Patent Document 5: Mol Cell Ther. 2016 Feb 27;4:3.

### Summary of the Invention

### [Problems to be solved by the Invention]

Under such situation, if causes of short plasma half-life and low bio-availability of FIX can be elucidated, and if FIX with extended plasma half-life compared to those of existing half-life-extended FIX formulations and with improved bio-availability can be created, it will allow prevention of bleeding by long dosage interval and subcutaneous administration. The present invention was achieved in view of the above circumstances. An objective of the present invention is to provide FIX with improved pharmacokinetics, methods for improving the pharmacokinetics of FIX, methods for controlling the pharmacokinetics of FIX, methods for producing FIX with improved pharmacokinetics, FIX-antibody complexes, and pharmaceutical compositions comprising the complex or FIX with improved pharmacokinetics as an active ingredient, etc. More specifically, an objective of the present invention is to elucidate the cause of short plasma half-life and low bio-availability of FIX, and to provide FIX with extended plasma half-life and/or improved bio-availability, methods for extending the plasma half-life and/or improving the bio-availability of FIX, methods for controlling the plasma half-life and/or bio-availability of FIX, methods for producing FIX with extended plasma half-life and/or improved bio-availability, FIX-antibody complexes, pharmaceutical compositions comprising the complex or FIX with improved plasma half-life and/or bio-availability as an active ingredient, and/or the pharmaceutical compositions for use in treating hemophilia B, and such.

### [Means for Solving the Problems]

To solve the above-mentioned problems, the present inventors attempted to elucidate the causes for the short plasma half-life and low bio-availability of FIX-Fc, and discovered that Gla residues included in the GLA domain of FIX-Fc are the causative factors. By deleting the GLA domain of FIX-Fc, the present inventors succeeded in extending the plasma half-life and improving the bio-availability. Furthermore, by inhibiting Gla-modification of FIX-Fc, the present inventors succeeded in extending the plasma half-life and improving the bio-availability. Furthermore, by using a GLA domain-recognizing antibody and inhibiting the function of Gla residues in worsening the pharmacokinetics, the present inventors succeeded in extending the plasma half-life and improving the bio-availability. The present invention is based on such findings and provides the following.
[1] A blood coagulation factor IX having either or both of an improved plasma half-life and an improved bio-availability, which comprises a modified GLA domain.
[2] The blood coagulation factor IX of [1], wherein the modification is one or more modifications selected from the group consisting of:
   (i) non-covalent bonding of a GLA-domain-recognizing antibody or an antibody fragment thereof to the GLA domain;
   (ii) reduced number of Gla residues in the GLA domain, in comparison to that of a native blood coagulation factor IX;
   (iii) either or both of deletion of one or more glutamic acid residues in the GLA domain and substitution of one or more glutamic acid residues in the GLA domain with another amino acid; and
   (iv) deletion of a part or all of the GLA domain.
[3] The blood coagulation factor IX of [2], wherein the antibody fragment is Fab, F(ab')2, or scFv.
[4] The blood coagulation factor IX of any one of [1] to [3], wherein the blood coagulation factor IX is a fusion protein formed with an FcRn-binding protein, or a fusion protein formed with an FcRn-binding protein and a GLA-domain-recognizing antibody or an antibody fragment thereof.
[5] A method for improving either or both of the plasma half-life and bio-availability of a blood coagulation factor IX, which comprises the step of modifying a GLA domain.
[6] The method of [5], wherein the modifying step is one or more steps selected from the group consisting of:
   (i) non-covalently binding a GLA-domain-recognizing antibody or an antibody fragment thereof to the GLA domain;
   (ii) reducing the number of Gla residues in the GLA domain, in comparison to that of a native blood coagulation factor IX;
   (iii) carrying out either or both of deletion of one or more glutamic acid residues in the GLA domain and substitution of one or more glutamic acid residues in the GLA domain with another amino acid; and
   (iv) deleting a part or all of the GLA domain.
[7] A method for controlling either or both of the plasma half-life and bio-availability of a blood coagulation factor IX, which comprises the step of modifying a GLA domain.
[8] The method of [7], wherein the modifying step is one or more steps selected from the group consisting of:
   (i) non-covalently binding a GLA-domain-recognizing antibody or an antibody fragment thereof to the GLA domain;
   (ii) reducing the number of Gla residues in the GLA domain, in comparison to that of a native blood coagulation factor IX;
   (iii) carrying out either or both of deletion of one or more glutamic acid residues in the GLA domain and substitution of one or more glutamic acid residues in the GLA domain with another amino acid; and
   (iv) deleting a part or all of the GLA domain.
[9] A method for producing a blood coagulation factor IX with either or both of an improved plasma half-life and an improved bio-availability of the blood coagulation factor IX, which comprises the step of modifying a GLA domain.
[10] The method of [9], wherein the modifying step is one or more steps selected from the group consisting of:
   (i) non-covalently binding a GLA-domain-recognizing antibody or an antibody fragment thereof to the GLA domain;
   (ii) reducing the number of Gla residues in the GLA domain, in comparison to that of a native blood coagulation factor IX;
   (iii) carrying out either or both of deletion of one or more glutamic acid residues in the GLA domain and substitution of one or more glutamic acid residues in the GLA domain with another amino acid; and
   (iv) deleting a part or all of the GLA domain.
[11] The method of [9] or [10], which further comprises the step of isolating a blood coagulation factor IX with a modified GLA domain.
[12] A complex formed between a blood coagulation factor IX and a GLA-domain-recognizing antibody or an antibody fragment thereof.
[13] A pharmaceutical composition which comprises the blood coagulation factor IX of any one of [1] to [4], a blood coagulation factor IX produced by the method of any one of [9] to [11], or the complex of [12] as an active ingredient.
[14] The pharmaceutical composition of [13], which is used for either or both of prevention and treatment of a FIX deficiency disease.
[15] The pharmaceutical composition of [14], wherein the FIX deficiency disease is hemophilia B.

### [Effects of the Invention]

FIX administered to a living body ordinarily disappears quickly, and maintaining it with a long plasma half-life had been difficult. For various biological molecules, modifications to extend the plasma half-life have been attempted. However, regarding FIX, when compared to other biological molecules, effects of extending the plasma half-life by modification were not yielded sufficiently due to problems inherent to FIX. The problems inherent to FIX, which make it difficult to maintain the effective plasma concentration of FIX for a long term, are resolved by the present invention. More specifically, the present invention elucidated that modification of the GLA domain of FIX enables to maintain the plasma concentration of FIX for a long term. The present invention also enabled extension of the plasma half-life of FIX-Fc, for which actions of extending the plasma half-life had not been sufficiently achieved as compared to IgG. The effects of the present invention are in discovering the problems inherent to FIX which precludes sufficient utilization of the plasma half-life-extending effect by Fc, and realizing means for making improvements on this problem.

Furthermore, in a preferred embodiment of the present invention, the invention not only succeeded in extending the plasma half-life, but also succeeded in maintaining a high plasma FIX-Fc concentration by subcutaneous administration. This result means that the bio-availability of FIX-Fc was improved. At present, most FIX formulations require intravenous injection, even when they are continuously administered for the purpose of prophylactic administration to prevent bleeding. Since the site of administration is limited for intravenous injection, the burden on the patients is large, particularly for FIX which presupposes long term administration. By improving the bio-availability of FIX, and for example, by enabling subcutaneous administration, the present invention greatly decreases the burden on patients.

### Brief Description of the Drawings

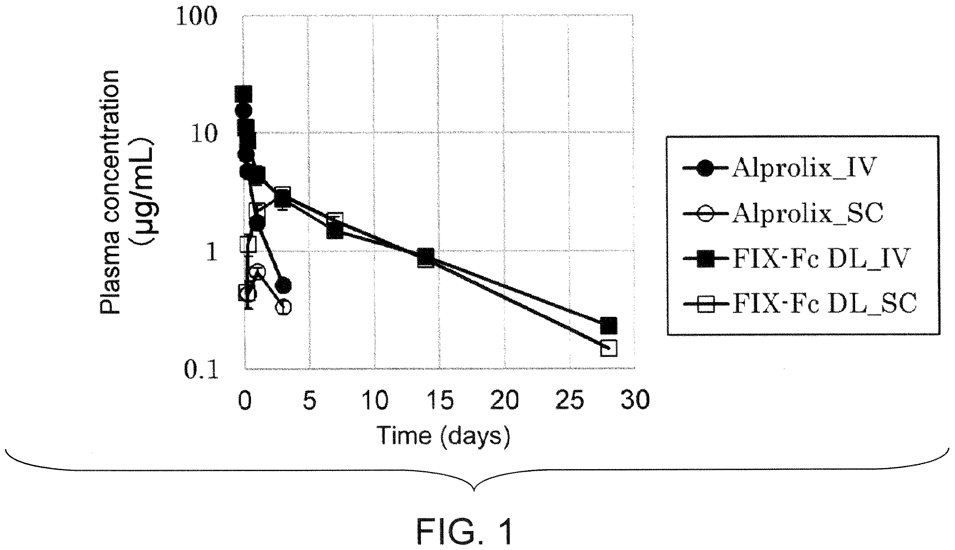

Fig. 1 is a graph showing the changes in plasma FIX concentrations after administering FIX-Fc (Alprolix) or GLA domain-less FIX-Fc to mice intravenously or subcutaneously. In the figure, the vertical axis indicates the plasma concentration of FIX (µg/mL) and the horizontal axis indicates the time (days) after administration.

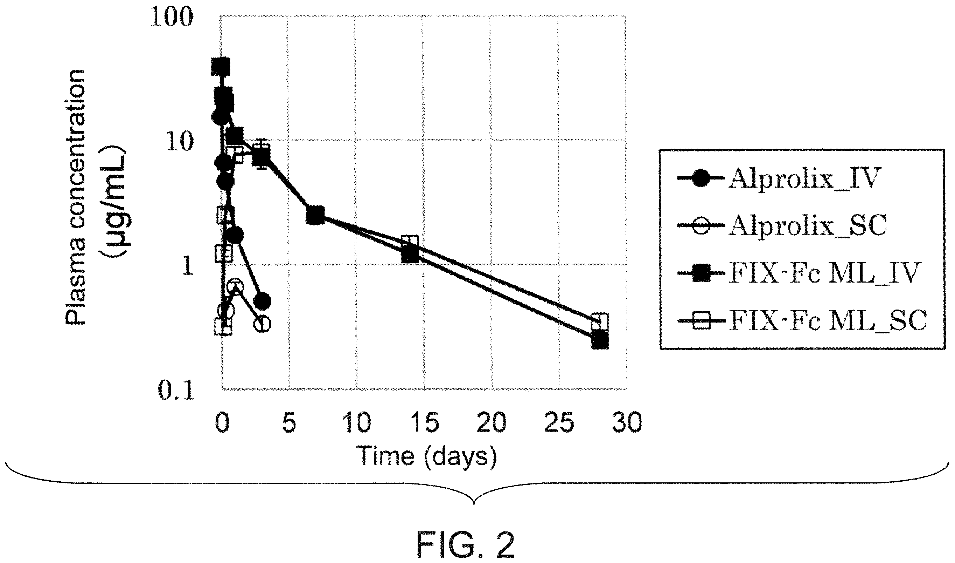

Fig. 2 is a graph showing the changes in plasma FIX concentrations after administering FIX-Fc (Alprolix) or Gla-modification-less FIX-Fc to mice intravenously or subcutaneously. In the figure, the vertical axis indicates the plasma concentration of FIX (µg/mL) and the horizontal axis indicates the time (days) after administration.

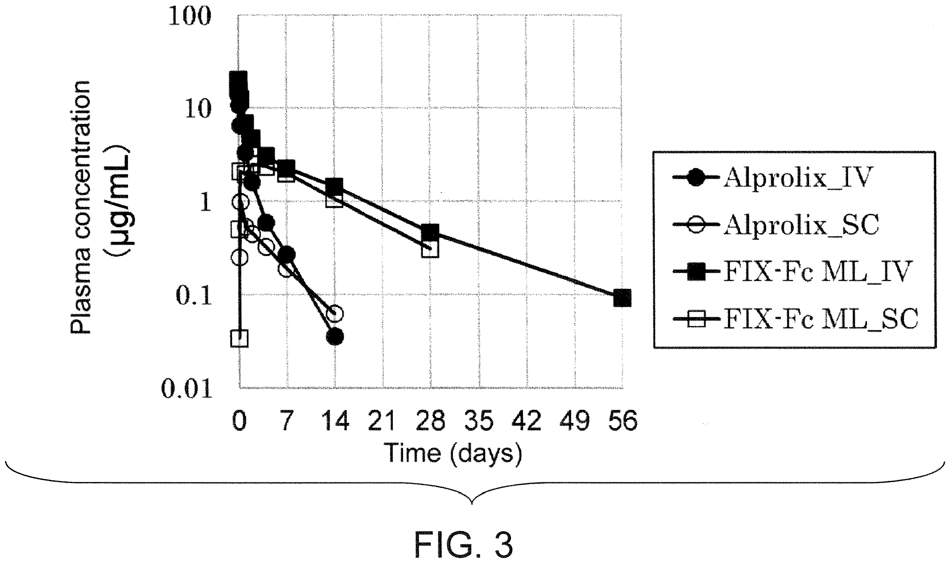

Fig. 3 is a graph showing the changes in plasma FIX concentrations after administering FIX-Fc (Alprolix) or Gla-modification-less FIX-Fc to cynomolgus monkeys intravenously or subcutaneously. In the figure, the vertical axis indicates the plasma concentration of FIX (µg/mL) and the horizontal axis indicates the time (days) after administration.

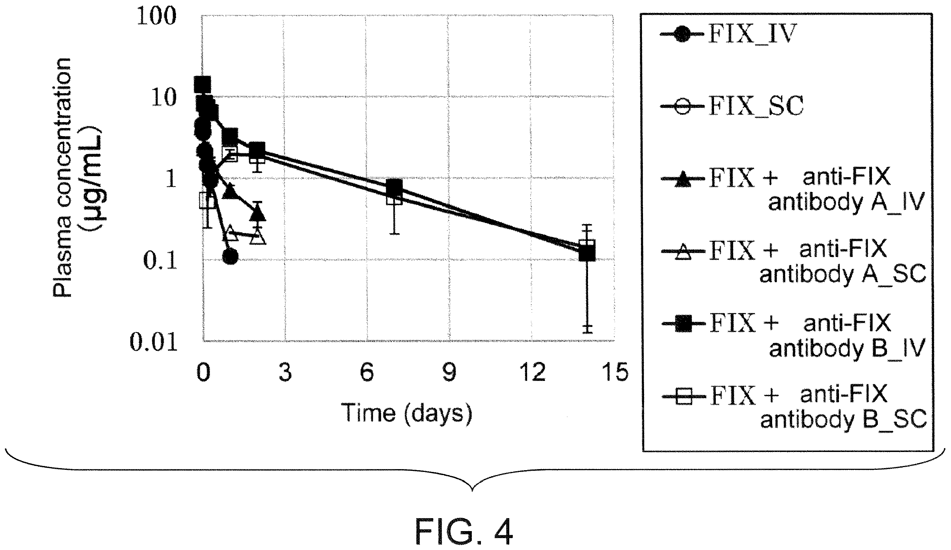

Fig. 4 is a graph showing the changes in plasma FIX concentrations after administering FIX alone, or FIX simultaneously with anti-FIX antibody A or with anti-FIX antibody B to mice intravenously or subcutaneously.

### Mode for Carrying Out the Invention

In one embodiment, the present invention relates to a FIX with either or both of an improved plasma half-life and an improved bio-availability, which comprises a modified GLA domain. In other words, the present invention relates to a modified FIX with either or both of a plasma half-life that is longer than that of a native FIX and bio-availability that is higher than that of a native FIX.

### FIX

FIX is not limited to human-derived FIX, and may be a FIX derived from humans, bovines, pigs, dogs, cats, or mice. In one aspect, FIX is a human FIX, and refers to a human FIX consisting of 415 amino acid residues, which is formed by the removal of the N-terminal signal sequence and the pro-peptide region consisting of 46 amino acids from the immature human FIX (SEQ ID NO: 1) consisting of 461 amino acid residues (see for example, UniProtKB/Swiss-Prot Accession P00740-1). Native human FIX is indicated by positions 47 to 461 in SEQ ID NO: 1. FIX includes any form of FIX which has the typical features of FIX. Generally, FIX contains a GLA domain (a region containing γ-carboxyglutamate residues), two EGF domains (human EGF homology domains), an activated peptide domain, and a C-terminal protease domain. However, it is not necessarily limited to FIX containing the above, and it may contain domains known in this technical field that are synonymous to these domains, or it may be fragments with partial deletions. FIX or sequence variants thereof have been cloned as described in U.S. Patent Nos. 4,770,999 and 7,700,734, and DNAs encoding human FIX have been isolated (see for example, Choo et al., Nature 299:178-180 (1982); and Kurachi et al., Proc. Natl. Acad. Sci., U.S.A. 79:6461-6464 (1982)). These known sequence variants include those carrying amino acid substitutions that enhance the FIX functions, but are not limited thereto. Herein below, when simply indicated as "FIX" this includes its sequence variants, unless specifically noted otherwise.

In a specific embodiment, the modified FIX of the present invention has either or both a plasma half-life that is longer than that of a native FIX and bio-availability that is higher than that of a native FIX, and for example, it comprises one or more modifications selected from the group consisting of:
(i) non-covalent bonding of a GLA-domain-recognizing antibody or an antibody fragment thereof to the GLA domain;
(ii) reduced number of Gla residues in the GLA domain, in comparison to that of a native FIX;
(iii) either or both of deletion of one or more glutamic acid residues in the GLA domain and substitution of one or more glutamic acid residues in the GLA domain with another amino acid; and
(iv) deletion of a part or all of the GLA domain.

### GLA domain

GLA domain is a region containing Gla residues (γ-carboxyglutamate residues), and may comprise the amino acid residues at positions 47 to 92 in SEQ ID NO: 1. FIX lacks positions 1 to 46 (the signal sequence) of SEQ ID NO: 1, and the GLA domain in FIX generally corresponds to positions 47 to 92 of SEQ ID NO: 1.

### Gla-modification (gamma-carboxylation)

Carboxylation of a glutamic acid residue in the presence of vitamin K to produce a Gla residue is called Gla-modification (gamma-carboxylation). FIX which undergoes vitamin K-dependent carboxylation is one of vitamin K-dependent coagulation factors.

In the present invention, the functions of FIX before the modification are desirably maintained after the modification in a modified FIX. Specifically, FIX is activated by the action of an activated factor XI (FXIa) or activated factor VII (FVIIa) complex, and produces activated factor IX (FIXa) possessing serine protease activity. Furthermore, FIXa together with activated factor VIII (FVIIIa) is involved in activation of factor X. Therefore, in the present invention a modified FIX is preferably able to constitute a FIX-involving reaction cascade similar to that described above. The functions of FIX can be verified by adding a modified FIX to blood samples or such which lack FIX from among the factors constituting blood coagulation, evaluating the blood coagulation ability and enzyme actions, and comparing the actions to those of an unmodified FIX. Alternatively, in an activated modified FIX generated by activation of a modified FIX, if the serine protease activity originally possessed by FIXa is maintained, one can observe that the modified FIX maintains the function of FIX. As a result of comparing the function, normally when the maintained function relative to the function of unmodified FIX is 70% or more, for example, 80% or more, preferably 90% or more, or 95% or more, one can consider that the function of FIX is maintained in the modified FIX.

### Plasma half-life (t1/2)

Plasma half-life refers to the time taken for a plasma drug concentration to decrease by one-half. In the present invention, whether the plasma half-life improved can be evaluated appropriately by carrying out pharmacokinetic (PK) examinations using, for example, mice, rats, rabbits, dogs, and monkeys. For example, by using *in vivo* kinetics analysis software WinNonlin (Pharsight) and following the attached instructions, appropriate evaluations can be carried out by Noncompartmental analysis.

According to plasma half-life evaluations, when extension of the half-life in comparison to that of a native FIX of, for example, 20% or more, preferably 30% or more, such as 40% or more, more specifically 50% or more, or even 60% or more, is observed, one can consider that the plasma half-life "improved" or "extended". In a preferred embodiment of the present invention, a plasma half-life exceeding at least 1.5 times or more, for example twice or more, or specifically three times or more compared to that of a native FIX can be achieved.

### Bio-availability (BA)

The term "bio-availability" indicates the rate and extent with which the pharmaceutical agents or other substances are absorbed or become available at the site of bioactivity after administration. It is an indicator of whether an extravascularly administered drug can reach the general blood circulation and exhibit its actions. It is evaluated as a value obtained by first dividing the area under the concentration curve for the administration method to be evaluated (for example, SC administration) (AUC_SC) by the dose (Dose_SC), and further dividing this by a value resulting from dividing the AUC for IV administration (AUC_IV) by the dose (Dose_IV). That is, it is calculated by (AUC_SC/Dose_SC)/(AUC_IV/Dose_IV). Since clearance (CL) is a value obtained by dividing the Dose by AUC, BA during SC administration can be calculated, for example, by dividing the CL for IV administration by the CL for SC administration. When absorption is poor and AUC cannot be evaluated sufficiently, BA is estimated from the maximum plasma concentration (Cmax). Bio-availability of a polypeptide can be tested by an *in vivo* pharmacokinetic method known in this field.

According to the FIX bio-availability evaluations, when the obtained bio-availability in comparison to that of a native FIX is, for example 20% or more, preferably 30% or more, such as 40% or more, more specifically 50% or more, or even 60% or more, one can consider that the bio-availability "improved" or "increased". In a preferred embodiment of the present invention, bio-availability exceeding at least 1.5 times or more, for example twice or more, or specifically three times or more compared to that of a native FIX can be achieved.

In another embodiment, the present invention relates to FIX with either or both of an improved plasma half-life and an improved bio-availability, which comprises a modified GLA domain, wherein the modification is one or more modifications selected from the group consisting of:
(i) non-covalent bonding of a GLA-domain-recognizing antibody or an antibody fragment thereof to the GLA domain;
(ii) reduced number of Gla residues in the GLA domain, in comparison to that of a native FIX;
(iii) either or both of deletion of one or more glutamic acid residues in the GLA domain and substitution of one or more glutamic acid residues in the GLA domain with another amino acid; and
(iv) deletion of a part or all of the GLA domain.

The "modified GLA domain" in the present invention includes a GLA domain carrying one or more modifications selected from the group consisting of (i) to (iv) described above, and as long as it provides FIX with improved plasma half-life and/or bio-availability, it is not necessarily limited thereto.

In a specific embodiment, a modified GLA domain refers to a GLA domain that is non-covalently bound with a GLA-domain-recognizing antibody or an antibody fragment thereof.

In one aspect, a GLA-domain-recognizing antibody or an antibody fragment thereof only needs to non-covalently bind to a GLA domain, and it may be an antibody that recognizes the entire GLA domain or an antibody fragment thereof, or an antibody that recognizes a part of the GLA domain or an antibody fragment thereof. In another aspect, a GLA-domain-recognizing antibody or an antibody fragment thereof is an antibody that specifically recognizes a GLA domain carrying Gla residues.

In another specific embodiment, modified GLA domain refers to a GLA domain with decreased number of Gla residues in the GLA domain in comparison to that of a native FIX. The number of Gla residues in comparison to that of a native FIX may be decreased by at least one residue, preferably two, three, four, five, or six residues, more preferably seven, eight, nine, or ten residues, or most preferably eleven or twelve residues.

In another specific embodiment, a modified GLA domain refers to a GLA domain subjected to either or both of a deletion of at least one or more glutamic acid residues in the GLA domain and substitution of at least one or more glutamic acid residues in the GLA domain with another amino acid. The deletion of glutamic acid residue or substitution of the same with another amino acid or both may be on at least one residue, preferably two, three, four, five, or six residues, more preferably seven, eight, nine, or ten residues, or most preferably eleven or twelve residues.

In another specific embodiment, a modified GLA domain refers to a GLA domain in which a part or all of the GLA domain has been deleted. Since the human FIX GLA domain is positions 47 to 92 of an immature FIX (SEQ ID NO: 1), for example, deletion of a part of a human FIX GLA domain may be a deletion of at least one residue or more, preferably five residues or more, ten residues or more, or more preferably 20 residues or more, 30 residues or more, or 40 residues or more in residues between positions 47 to 92. Furthermore, deletion of all of the GLA domain refers to deletion of the amino acid residues at positions 47 to 92 of SEQ ID NO: 1.

As long as the objective of the present invention is accomplished, the modified GLA domain may include other amino acid residue substitutions, additions, deletions, and such.

For example, the GLA domain in human FIX comprises twelve glutamic acid residues (Glu/E). These Glu/E correspond to positions 53, 54, 61, 63, 66, 67, 72, 73, 76, 79, 82, and 86 of immature FIX (SEQ ID NO: 1) (which are positions 7, 8, 15, 17, 20, 21, 26, 27, 30, 33, 36, and 40 of FIX, respectively). Therefore, by deleting or substituting any one or more of these Glu/E residues, a "modified GLA domain" of the present invention can be obtained. In the present invention, deleting or substituting a plurality of Glu/E residues yields FIX in which Glu/E residue deletion and Glu/E residue substitution to another amino acid residue are mixed in one molecule.

For example, the GLA domain in human FIX comprises twelve glutamic acid residues (Glu/E). These Glu/E correspond to positions 53, 54, 61, 63, 66, 67, 72, 73, 76, 79, 82, and 86 of immature FIX (SEQ ID NO: 1) (which are positions 7, 8, 15, 17, 20, 21, 26, 27, 30, 33, 36, and 40 of FIX, respectively). Therefore, by deleting or substituting any of these Glu/E residues, a modified FIX of the present invention can be obtained.

### Polypeptides

In the present invention, polypeptides ordinarily refer to proteins and peptides having a length of approximately ten amino acids or longer. Furthermore, they are ordinarily peptides derived from living beings, but are not particularly limited thereto, and may be, for example, polypeptides comprising an artificially designed sequence. They may also be any of naturally-occurring polypeptides, synthetic polypeptides, recombinant polypeptides, and such. Furthermore, fragments of the above-mentioned polypeptides are included in the polypeptides of the present invention.

In the present invention, the polypeptides may be isolated polypeptides. Isolated generally refers to the state where the polypeptides are substantially homogeneous and do not include other contaminants. In the present invention, the polypeptides preferably do not include biological components other than polypeptides at detectable levels. When polypeptide purity is, for example 80% or 90%, preferably 95%, more preferably 98%, and even more preferably 99% or more, it is referred to as not containing contaminants. The purity of the polypeptides can be determined by known methods such as electrophoresis.

In one aspect, the present invention provides a fusion protein formed between FIX and an FcRn-binding protein, or a fusion protein formed by FIX, an FcRn-binding protein, and a GLA domain-recognizing antibody or an antibody fragment thereof.

Herein, the FcRn-binding protein may be, for example IgG or albumin, but is not limited thereto as long as the protein can bind (has binding activity or affinity) to FcRn. While not particularly limited to the following, preferably, the FcRn-binding protein in the present invention is a human IgG, a human IgG heavy chain, a human IgG Fc, a part of a human IgG Fc, or modified forms thereof, and as long as it is a protein that may bind to FcRn, it is included in the FcRn-binding protein of the present invention.

Herein, the term "Fc" is used to define a C-terminal region of an immunoglobulin heavy chain which comprises at least a part of the constant region. This term includes Fc of a native sequence and a mutant Fc. In one embodiment, Fc is a human IgG Fc, and the IgG may be any of IgGI, IgG2, IgG3, and IgG4. In one embodiment, human IgG Fc comprises a hinge, CH2, and CH3. In one embodiment, human IgG Fc extends from Cys226 or from Pro230 to the carboxyl terminal of the heavy chain. However, the lysine (Lys447) or glycine-lysine (Gly446-Lys447) at the C terminus of Fc may or may not be present. Herein, unless particularly stated otherwise, the amino acid residues in Fc or the constant region are numbered according to the EU numbering system (also called EU index) described in Kabat et al., Sequences of Proteins of Immunological Interest, 5th Ed. Public Health Service, National Institutes of Health, Bethesda, MD 1991. The terms "antibody", "immune globulin", and "immunoglobulin" are interchangeable and are used in a broad sense.

Herein, the fusion protein formed between a FIX and an FcRn-binding protein refers to a protein in which a FIX and an FcRn-binding protein are fused (covalently bound). In one embodiment, a fusion protein formed with an FcRn-binding protein is an assembly formed from FIX-hinge-CH2-CH3 and hinge-CH2-CH3. In the present invention, a fusion protein formed with Fc may be denoted as "-Fc".

Herein, a fusion protein formed from a FIX, an FcRn-binding protein, and a GLA domain-recognizing antibody or an antibody fragment thereof comprises a FIX, an FcRn-binding protein, and a GLA domain-recognizing antibody or an antibody fragment thereof, and refers to a protein in which a FIX is fused (covalently bound) to either or both of an FcRn-binding protein and a GLA domain-recognizing antibody or an antibody fragment thereof. For example, it may be a fusion protein in which a FIX is covalently bound with an FcRn-binding protein, and furthermore, the FcRn-binding protein is covalently bound with a GLA domain-recognizing antibody or an antibody fragment thereof. In one embodiment the fusion protein is a fusion protein in which a FIX and a GLA domain-recognizing antibody or an antibody fragment thereof are each covalently bound with Fc. Furthermore, for example, it may be a fusion protein in which a FIX is covalently bound with a GLA domain-recognizing antibody or an antibody fragment thereof, and further, the GLA domain-recognizing antibody or an antibody fragment thereof is covalently bound to an FcRn-binding protein. Furthermore, it may be a fusion protein in which FIX is covalently bound to each of an FcRn-binding protein, and a GLA domain-recognizing antibody or an antibody fragment thereof.

The DNA encoding FIX can be produced based on a DNA encoding the above-mentioned known human FIX. Amino acid-residue substitutions, insertions, or deletions can be performed by methods known to those skilled in the art.

When substituting an amino acid residue, substitution to another amino acid residue may be performed, for example, with the objective of causing changes to the following:
(a) polypeptide backbone structure in the sheet structure or helical structure region;
(b) charges or hydrophobicity in the target region; or
(c) size of the side chain.

Amino acid residues are classified into the following groups based on the general properties of the side chains:
(1) hydrophobic: norleucine, met, ala, val, leu, ile;
(2) neutral hydrophilic: cys, ser, thr, asn, gln;
(3) acidic: asp, glu;
(4) basic: his, lys, arg;
(5) residues that affect the chain orientation: gly, pro; and
(6) aromatic: trp, tyr, phe.

Substitution of amino acid residues within each of these groups is called conservative substitution; on the other hand, substitution of amino acid residues between different groups is referred to as non-conservative substitution. Substitutions in the present invention may be conservative substitutions or non-conservative substitutions, or a combination of conservative substitutions and non-conservative substitutions. In conservative substitution, an amino acid residue is substituted with that having properties similar to the original amino acid residue; therefore, effects of the substitution on the biological activity and physicochemical properties of the entire peptide can be made small.

Amino acid sequence alterations are produced by various methods known in this field. Such methods include the site-directed mutagenesis method (Hashimoto-Gotoh, T, Mizuno, T, Ogasahara, Y, and Nakagawa, M. (1995) An oligodeoxyribonucleotide-directed dual amber method for site-directed mutagenesis. Gene 152, 271-275, Zoller, MJ, and Smith, M. (1983) Oligonucleotide-directed mutagenesis of DNA fragments cloned into M13 vectors. Methods Enzymol. 100, 468-500, Kramer, W, Drutsa, V, Jansen, HW, Kramer, B, Pflugfelder, M, and Fritz, HJ (1984) The gapped duplex DNA approach to oligonucleotide-directed mutation construction. Nucleic Acids Res. 12, 9441-9456, Kramer W, and Fritz HJ (1987) Oligonucleotide-directed construction of mutations via gapped duplex DNA Methods. Enzymol. 154, 350-367, Kunkel, TA (1985) Rapid and efficient site-specific mutagenesis without phenotypic selection. Proc Natl Acad Sci U S A. 82, 488-492), the PCR mutation method, and the cassette mutation method, but are not limited thereto.

FIX can be produced using gene recombination techniques known to those skilled in the art. Specifically, a DNA encoding FIX is constructed, this is introduced into an expression vector, and then this is expressed in appropriate host cells.

Examples of the vectors include M13-series vectors, pUC-series vectors, pBR322, pBluescript, and pCR-Script. In addition to these vectors, pGEM-T, pDIRECT, pT7, and the like can also be used when the objective is subcloning and excising cDNA.

Particularly, expression vectors are useful when using the vectors for the purpose of producing FIX. For example, when the host is *Escherichia coli* such as JM109, DH5α, HB101, and XL1-Blue, the expression vectors indispensably have a promoter that permits efficient expression in *E. coli,* for example, lacZ promoter, araB promoter, or T7 promoter. Other than the above-mentioned vectors, examples of such vectors include pGEX-5X-1, pEGFP, and pET.

The vectors may contain signal sequences for polypeptide secretion. As a signal sequence for polypeptide secretion, a pelB signal sequence may be used when the polypeptide is produced in the *E. coli* periplasm. The vector can be introduced into host cells by a lipofectin method, a calcium phosphate method, a DEAE-Dextran method, and the like.

In addition to expression vectors for *E. coli,* vectors for producing polypeptides include expression vectors derived from mammals (for example, pcDNA3 (manufactured by Invitrogen), pEGF-BOS, pEF, and pCDM8), expression vectors derived from insect cells (for example, the "Bac-to-BAC baculovirus expression system" (manufactured by Gibco-BRL) and pBacPAK8), expression vectors derived from plants (for example, pMH1 and pMH2), expression vectors derived from animal viruses (for example, pHSV, pMV, and pAdexLcw), expression vectors derived from retroviruses (for example, pZIPneo), expression vectors derived from yeasts (for example, "Pichia Expression Kit" (manufactured by Invitrogen), pNV11, and SP-Q01), and expression vectors derived from *Bacillus subtilis* (for example, pPL608 and pKTH50), for example.

When the objective is expression in animal cells such as CHO, COS, and NIH3T3 cells, the expression vectors must have a promoter required for expression in cells, for example, SV40 promoter, MMTV-LTR promoter, EF1α promoter, CAG promoter, and CMV promoter, and more preferably, the vectors have a gene for selecting transformed cells (for example, a drug resistance gene that allows discrimination using a pharmaceutical agent (neomycin, G418, or such)). Vectors with such characteristics include pMAM, pDR2, pBK-RSV, pBK-CMV, pOPRSV, and pOP13, for example.

When producing FIX, cells that express any one or a combination of two or more of vitamin K epoxide reductase (VKOR), vitamin K-dependent γ-glutamyl carboxylase (GGCX), and furin, may be used. GGCX is an enzyme that promotes Gla-modification, and vitamin K, which is its coenzyme, is produced by VKOR. GGCX binds to the propeptide region of FIX and promotes Gla-modification. Furin cleaves the propeptide region. As confirmed in the Examples, co-expression of FIX with VKOR and furin leads to suppression of vitamin K-dependent Gla-modification of FIX, and as a result, the level of Gla-modification of the GLA domain decreases. As a result, even when FIX itself is expressed as an intact native amino acid sequence, it can be collected as a polypeptide with decreased level of Gla-modification.

Any one or two or more of VKOR, GGCX, and furin can be inserted into the same vector or into separate vectors. Furthermore, any one or two or more of VKOR, GGCX, and furin can be inserted into the same vector as that of FIX. Furthermore, VKOR, GGCX, and furin can be expressed simultaneously, or they may be expressed sequentially.

The DNA sequence of human GGCX (GenBank: KJ891238.1, Wu, S.-M., Cheung, W.-F., Frazier, D., Stafford, D. W. Science 254: 1634-1636, 1991) is described in U.S. Patent No. 5,268,275. The DNA of human VKOR was described in 2004 (Li et al., Nature 427:541-543, 2004; and Rost et al., Nature 427:537-541, 2004).

Furin is a calcium-dependent paired basic amino acid converting enzyme (PACE), and specifically cleaves the peptide bond C-terminal to arginine in a specific sequence. Furin is involved in the maturation of several human proproteins. DNA encoding furin and the amino acid sequence of furin are already known (EMBO J. 5: 2197-2202, 1986).

In addition, when the objective is stable gene expression and gene copy number amplification in cells, methods that can be used include the method of introducing into CHO cells deficient in a nucleic acid synthesis pathway a vector that carries a DHFR gene which compensates for the deficiency (for example, pCHOI), and amplifying the gene using methotrexate (MTX). Alternatively, when the objective is transient gene expression, methods that can be used include the method of transforming COS cells that carry a gene expressing SV40 T antigen on their chromosome, using a vector with an SV40 replication origin (pcD and such).

Replication origins derived from polyoma virus, adenovirus, bovine papilloma virus (BPV), and such can also be used. To amplify gene copy number in host cells, the expression vectors may carry selection markers such as aminoglycoside transferase (APH) gene, thymidine kinase (TK) gene, *E. coli* xanthine-guanine phosphoribosyltransferase (Ecogpt) gene, and dihydrofolate reductase (dhfr) gene.

FIX can be collected, for example by culturing the transformed cells, and then isolating FIX from inside these molecularly transformed cells or from the culture medium. FIX can be isolated and purified by appropriately combining methods such as centrifugation, ammonium sulfate fractionation, salting-out, ultrafiltration, affinity chromatography, ion exchange chromatography, and gel filtration chromatography.

The antibodies used in the present invention are not particularly limited, but are preferably monoclonal antibodies. The monoclonal antibodies include not only monoclonal antibodies derived from animals such as humans, mice, rats, hamsters, rabbits, sheep, camels, and monkeys, but also include artificially modified gene recombinant antibodies such as chimeric antibodies, humanized antibodies, and bispecific antibodies. Recombinant antibodies can be obtained by cloning DNAs encoding the antibodies from hybridomas or from antibody-producing cells, such as sensitized lymphocytes that produce antibodies, inserting them into suitable vectors, and then introducing them into hosts (host cells) to produce the antibodies.

Methods for obtaining human antibodies are already known. For example, transgenic animals carrying the entire repertoire of human antibody genes can be immunized with desired antigens to obtain desired human antibodies (see International Publication No. WO 93/12227, WO 92/03918, WO 94/02602, WO 94/25585, WO 96/34096, and WO 96/33735).

Genetically modified antibodies can be produced using known methods. Specifically, for example, chimeric antibodies are antibodies that comprise H chain and L chain variable regions of an antibody of the immunized animal, and H chain and L chain constant regions of a human antibody. Chimeric antibodies can be obtained by linking DNAs encoding the variable regions of the antibody derived from the immunized animal, with DNAs encoding the constant regions of a human antibody, inserting this into an expression vector, and then introducing it into hosts to produce the antibodies.

Humanized antibodies are genetically modified antibodies also referred to as "reshaped" human antibodies. A humanized antibody is constructed by grafting the CDRs of an antibody derived from an immunized animal to the CDRs of a human antibody. Conventional genetic recombination techniques for such purposes are also known (see European Patent Application Publication No. EP 239400; International Publication No. WO 96/02576; Sato K et al., Cancer Research 1993, 53: 851-856; International Publication No. WO 99/51743).

Antibody fragments used in the present invention are not particularly limited, and refer to a molecule other than a complete antibody which molecule comprises a portion of the complete antibody that binds to an antigen recognized by the complete antibody. Examples of antibody fragments include but are not limited to Fv, Fab, Fab', Fab'-SH, F(ab')2, diabody, linear antibody, single-chain antibody molecule (for example, scFv), and multispecific antibody formed from antibody fragments.

Modified antibodies include, for example, antibodies bound to various molecules such as polyethylene glycol (PEG). Antibodies used in the present invention include these modified antibodies. The substances bound to the modified antibodies used in the present invention are not limited. To afford such modified antibodies, chemical modifications can be carried out on the obtained antibodies. These methods are already established in this field.

Alternatively, in another embodiment of the present invention, a GLA domain can be modified by non-covalently binding a GLA-domain-recognizing antibody or an antibody fragment thereof to FIX. A GLA-domain-recognizing antibody or an antibody fragment thereof may be an antibody that specifically recognizes a GLA domain containing Gla residues. Herein below, an antibody or an antibody fragment thereof that non-covalently binds to a GLA domain and modifies this portion is called "a GLA-domain-modifying antibody or an antibody fragment thereof'. Specifically, a GLA-domain-modifying antibody or an antibody fragment thereof can be contacted with FIX in advance to obtain a GLA-domain-modified FIX, and this FIX can be administered. Alternatively, a GLA-domain-modifying antibody or an antibody fragment thereof can be administered together with FIX, and the administered FIX can be modified *in vivo.*

Furthermore, the present invention provides a method for producing a FIX comprising a modified GLA domain, wherein the method comprises the steps of non-covalently binding a GLA-domain-modifying antibody or an antibody fragment thereof to FIX, and then isolating the FIX in which the GLA domain has been modified by the antibody. Alternatively, the present invention relates to a method for either or both of treating and preventing a FIX deficiency disease, which comprises the steps of covalently binding a GLA-domain-modifying antibody or an antibody fragment thereof to FIX in advance, and then administering the GLA-domain-modified FIX to a subject. In addition, the present invention relates to a method for either or both of treating and preventing a FIX deficiency disease, which comprises the step of administering to a subject a GLA-domain-modifying antibody or an antibody fragment thereof together with FIX.

In the present invention "FIX deficiency disease" refers to a disease in which the condition where plasma FIX level is less than the normal range due to some kind of etiology is ongoing. Herein, plasma FIX level includes both the protein level and the biological activity level of FIX. Therefore, when either the protein level or the biological activity level of FIX is less than the normal range, that condition can be referred to as FIX deficiency disease. Generally, the plasma FIX activity level of a healthy adult is evaluated by the activated partial thromboplastin time (APTT). Therefore, a level below this range is determined as FIX deficiency disease. A representative FIX deficiency disease is hemophilia B.

GLA-domain-modifying antibodies or antibody fragments thereof can be obtained by known methods. That is, necessary antibodies can be obtained by using as immunogen, a FIX comprising a GLA domain or a polypeptide comprising at least a portion of that GLA domain, or a FIX comprising a GLA domain comprising Gla residues or a polypeptide comprising at least a portion of that GLA domain. For the antibodies or antibody fragments thereof, GLA domain-modifying antibodies or antibody fragments thereof can be selected by confirming as necessary their specificity to the GLA domain or their specificity to the GLA domain comprising Gla residues. The FIX-modifying ability of the GLA domain-modifying antibodies or antibody fragments thereof can be found out by actually administering them with the modified FIX to model animals for pharmacokinetic evaluation, and evaluating the plasma pharmacokinetics. In the present invention, GLA domain-modifying antibodies or antibody fragments thereof are preferably monoclonal antibodies. The GLA domain-modifying antibody fragments of the present invention may be Fab, F(ab')2, scFv, or such, as long as their ability to modify FIX is maintained. Alternatively, Fc that matches the animal receiving the administration can be combined for chimerization. For example, when the objective is administration to humans, human Fc can be used. Furthermore, by recombination to a human sequence while keeping the variable region CDRs, GLA domain-modifying antibodies or antibody fragments thereof can be humanized.

To evaluate the non-covalent binding of the antibodies or antibody fragments thereof of this description to a GLA domain or Gla residues, methods generally known to those skilled in the art such as SPR and ELISA in the presence or absence of calcium can be used for verification.

In another aspect, the present invention provides a method for improving the plasma half-life and/or bio-availability of FIX, wherein the method comprises a step of modifying a GLA domain. Alternatively, the present invention provides a method for improving both or either one of the plasma half-life and bio-availability of FIX, wherein the method comprises a step of modifying a GLA domain.

In another aspect, the present invention relates to a method for improving both or either one of the plasma half-life and bio-availability of FIX, wherein the method comprises a step of modifying a GLA domain, and the modifying step is one or more steps selected from the group consisting of:
(i) non-covalently binding a GLA-domain-recognizing antibody or an antibody fragment thereof to the GLA domain;
(ii) reducing the number of Gla residues in the GLA domain, in comparison to that of a native FIX;
(iii) deleting one or more glutamic acid residues in the GLA domain and/or substituting one or more glutamic acid residues in the GLA domain with another amino acid; and
(iv) deleting a part or all of the GLA domain.

In another aspect, the present invention provides a method for controlling the plasma half-life and/or bio-availability of FIX, wherein the method comprises a step of modifying a GLA domain. Alternatively, the present invention provides a method for controlling both or either one of the plasma half-life and bio-availability of FIX, wherein the method comprises a step of modifying a GLA domain.

In another aspect, the present invention relates to a method for controlling both or either one of the plasma half-life and bio-availability of FIX, wherein the method comprises a step of modifying a GLA domain, and the modifying step is one or more steps selected from the group consisting of:
(i) non-covalently binding a GLA-domain-recognizing antibody or an antibody fragment thereof to the GLA domain;
(ii) reducing the number of Gla residues in the GLA domain, in comparison to that of a native FIX;
(iii) deleting one or more glutamic acid residues in the GLA domain and/or substituting one or more glutamic acid residues in the GLA domain with another amino acid; and
(iv) deleting a part or all of the GLA domain.

In the present invention, controlling the plasma half-life of FIX ordinarily refers to extension of plasma half-life. In this sense, the present invention relates to a method for extending the plasma half-life of FIX, which comprises a step of modifying a GLA domain. Similarly, improvement of "bio-availability" in the present invention ordinarily refers to increasing the bio-availability. Therefore, the present invention relates to a method for increasing the bio-availability of FIX, which comprises a step of modifying the GLA domain.

Furthermore, in another aspect, the present invention provides a method for producing FIX with improved plasma half-life and/or bio-availability of the FIX, wherein the method comprises a step of modifying a GLA domain. Alternatively, the present invention provides a method for producing FIX with improvement in either one or both of FIX plasma half-life and bio-availability, wherein the method comprises the step of modifying a GLA domain.

In another aspect, the present invention relates to a method for producing a FIX with improvement in either one or both of FIX plasma half-life and bio-availability, wherein the method comprises a step of modifying a GLA domain, and the modifying step is one or more steps selected from the group consisting of:
(i) non-covalently binding a GLA-domain-recognizing antibody or an antibody fragment thereof to the GLA domain;
(ii) reducing the number of Gla residues in the GLA domain, in comparison to that of a native FIX;
(iii) deleting one or more glutamic acid residues in the GLA domain and/or substituting one or more glutamic acid residues in the GLA domain with another amino acid; and
(iv) deleting a part or all of the GLA domain.

In the present invention, a method for producing FIX with improvement in either one or both of FIX plasma half-life and bio-availability, may comprise a step of isolating a FIX in which the GLA domain has been modified.

A complex formed between a FIX and a GLA domain-recognizing antibody or an antibody fragment thereof in the present invention may be a non-covalently bound conjugate formed by specific recognition of a FIX by a GLA domain-recognizing antibody or an antibody fragment thereof, or a connected body formed by connection of a FIX with a GLA domain-recognizing antibody or an antibody fragment thereof through a linker or such. Furthermore, in such a connected body, a GLA domain-recognizing antibody or an antibody fragment thereof may specifically recognize a FIX and non-covalently bind therewith. The complex may be an Fc fusion protein.

In another aspect, the present invention provides a pharmaceutical composition which comprises the FIX or complex as an active ingredient. Pharmaceutical compositions of the present invention can be formulated by known methods by introducing a pharmaceutically acceptable carrier in addition to the FIX or complex. Namely, the present invention provides a method for producing a pharmaceutical composition, which comprises the step of formulating or mixing a pharmaceutically acceptable carrier with the FIX or complex. Alternatively, the present invention provides a pharmaceutical composition for either or both of treating and preventing a FIX deficiency disease, which comprises the FIX or complex. Furthermore, the present invention relates to use of the FIX or complex in the manufacture of pharmaceutical compositions for either or both of treatment and prevention of a FIX deficiency disease. The present invention also relates to use of the FIX or complex in either or both of treatment and prevention of a FIX deficiency disease.

For example, the present invention can be used parenterally, in the form of injections of sterile solutions or suspensions prepared with water or other pharmaceutically acceptable liquid. For example, formulations may be made by appropriately combining with a pharmaceutically acceptable carrier or medium, specifically, sterile water, physiological saline, vegetable oil, emulsifier, suspension, surfactant, stabilizer, flavoring agent, excipient, vehicle, preservative, binder, or such, and mixed in unit, amount and form required for realization of generally accepted pharmaceutical preparations. Specific examples of the carriers include light anhydrous silicic acid, lactose, crystalline cellulose, mannitol, starch, carmellose calcium, carmellose sodium, hydroxypropyl cellulose, hydroxypropyl methylcellulose, polyvinylacetal diethylaminoacetate, polyvinylpyrrolidone, gelatin, medium-chain fatty acid triglyceride, polyoxyethylene hydrogenated castor oil 60, white soft sugar, carboxymethyl cellulose, corn starch, inorganic salt, and such. The amount of the active ingredient in such a formulation is adjusted so that an appropriate content within the specified range can be obtained.

Sterile compositions for injection can be prescribed using vehicles such as distilled water for injection, according to standard formulation practice.

Aqueous solutions used for injection include, for example, physiological saline and isotonic solutions containing glucose or other adjuvants, such as D-sorbitol, D-mannose, D-mannitol, and sodium chloride. These can be used in combination with suitable solubilizers such as alcohol, specifically ethanol, polyalcohols such as propylene glycol and polyethylene glycol, and non-ionic surfactants such as Polysorbate 80™ and HCO-50.

Oily liquids include sesame oils and soybean oils, and can be combined with solubilizers such as benzyl benzoate or benzyl alcohol. These may also be formulated with buffers, for example, phosphate buffers or sodium acetate buffers; analgesics, for example, procaine hydrochloride; stabilizers, for example, benzyl alcohol or phenol; or antioxidants. The prepared injections are typically loaded into appropriate ampules.

The administration is preferably carried out parenterally, and specifically includes dosage forms for injection, intranasal administration, intrapulmonary administration, and percutaneous administration. For example, dosage forms for injections include systemic or local administration by intravenous injection, intramuscular injection, intraperitoneal injection, or subcutaneous injection.

Furthermore, the method of administration can be appropriately selected according to the age and symptoms of the patient. A single dosage of the pharmaceutical composition of the present invention can be selected, for example, from the range of 0.0001 mg to 1,000 mg per kg of body weight. Alternatively, the dosage may be selected, for example, in the range of 0.001 to 100,000 mg/body of a patient. However, the dosage is not necessarily limited to these values. The dosage and method of administration vary depending on the patient's body weight, age, and symptoms, and can be appropriately selected by those skilled in the art.

In one aspect, the pharmaceutical composition of the present invention is used for preventing and/or treating diseases that develop and/or progress due to reduction or deficiency in activity of FIX and/or FIXa. In a specific embodiment, more specifically, a pharmaceutical composition of the present invention is used for preventing and/or treating hemophilia B.

The present invention improved either one or preferably both of the blood pharmacokinetics and bio-availability of FIX. First, through improvement of the pharmacokinetics of FIX, the blood half-life of FIX became extended two to three-fold compared to that of a FIX-Fc fusion protein. This result means that the administration interval can be extended two to three-folds of that required for FIX-Fc. Therefore, the present invention provides a method for treating a subject, which comprises administering a modified FIX of the present invention to a subject in need of a FIX administration, wherein the modified FIX of the present invention is administered at an administration interval that is at least twice or preferably three times or more the administration interval needed for FIX-Fc. Alternatively, the present invention provides a pharmaceutical composition comprising a modified FIX of the present invention for administration to a subject in need of a FIX administration, wherein the composition is for administering the modified FIX of the present invention at an administration interval that is at least twice or preferably three times or more the administration interval needed for FIX-Fc. Furthermore, the present invention relates to use of a modified FIX of the present invention for treating a subject in need of a FIX administration, wherein the use is for administering the modified FIX of the present invention at an administration interval that is at least twice or preferably three times or more the administration interval needed for FIX-Fc. In another embodiment, the present invention provides use of a modified FIX of the present invention in the manufacture of pharmaceutical compositions for treating a subject in need of a FIX administration, wherein the use is for producing a pharmaceutical composition for administering to the subject the modified FIX of the present invention at an administration interval that is at least twice or preferably three times or more the administration interval needed for FIX-Fc.

Next, the present invention yielded improvement in bio-availability of FIX. Because of this improvement of bio-availability, subcutaneous administration of a modified FIX of the present invention to a subject in need of a FIX administration can yield therapeutic effects equivalent to when a FIX-Fc fusion protein is administered intravenously. Therefore, the present invention provides a method for treating a subject, which comprises administering a modified FIX of the present invention to a subject in need of a FIX administration, wherein the modified FIX of the present invention is subcutaneously administered to the subject. Alternatively, the present invention provides a pharmaceutical composition comprising a modified FIX of the present invention for administration to a subject in need of a FIX administration, wherein the composition is for subcutaneous administration of the modified FIX of the present invention to the subject. Furthermore, the present invention relates to use of a modified FIX of the present invention for treating a subject in need of a FIX administration, wherein the use is for subcutaneous administration of the modified FIX of the present invention to the subject. In another embodiment, the present invention also provides use of a modified FIX of the present invention in the manufacture of pharmaceutical compositions for treating a subject in need of a FIX administration, wherein the use is for producing a pharmaceutical composition for subcutaneous administration of the modified FIX of the present invention to the subject.

The three-letter and one-letter expressions of the amino acids used herein are as follows.
Alanine: Ala: A
Arginine: Arg: R
Asparagine: Asn: N
Aspartic acid: Asp: D
Cysteine: Cys: C
Glutamine: Gln: Q
Glutamic acid: Glu: E
Glycine: Gly: G
Histidine: His: H
Isoleucine: lie: I
Leucine: Leu: L
Lysine: Lys: K
Methionine: Met: M
Phenylalanine: Phe: F
Proline: Pro: P
Serine: Ser: S
Threonine: Thr: T
Tryptophan: Trp: W
Tyrosine: Tyr: Y
Valine: Val: V

All prior art documents cited herein are incorporated by reference into this description.

### Examples

### [Example 1] Effects of the GLA domain on pharmacokinetics of FIX-Fc in mice

### 1-1. Preparation of GLA domain-deficient FIX-Fc

The half-life of FIX-Fc (Alprolix) which is a fusion protein formed by FIX and human IgG1 Fc is approximately 82 hours, and compared to FIX having a half-life of approximately 18 hours, the half-life is extended by approximately four to five times; however, when compared to the half-life of a monoclonal antibody carrying the same human IgG1 Fc, which is two to three weeks, the half-life is considerably short.

The cause for this short half-life has not yet been reported. Accordingly, we set up a hypothesis that this short half-life is caused by the GLA domain of FIX in the FIX-Fc protein. Then, we prepared FIX-Fc molecules in which their GLA domain is deleted (FIX-Fc GLA Domain Less; FIX-Fc DL). Herein below, human FIX was used for FIX unless particularly stated otherwise.

FIX-Fc DL was expressed as described below. Expression vectors encoding FIX-DL-hinge-CH2-CH3 (SEQ ID NO: 2) and hinge-CH2-CH3 (SEQ ID NO: 3) were prepared by a method known to those skilled in the art, and gene transfection was performed using a transient expression system of Expi 293 (Life Technologies). On the second day after gene transfection, vitamin K1 (Sigma-Aldrich) was added together with an enhancer (Life technologies), and the culture supernatant was collected on the fifth day. From the obtained culture supernatant, FIX-Fc DL was purified by a method known to those skilled in the art using HiTrap Heparin HP 1 mL (GE Healthcare) and Superdex200 10/300 increase (GE Healthcare), and the concentration was calculated using the PACE method.

### 1-2. Pharmacokinetic evaluation of Alprolix and FIX-Fc DL in mice

Pharmacokinetic tests (PK tests) using mice were performed by the following method. Alprolix (product name; a fusion protein formed between FIX and Fc) or FIX-Fc DL was administered through the tail vein (IV) or under the dorsal skin (SC) of mice (C57BL/6J mouse, Charles River Japan) in a single dose at 2 mg/kg. Blood was collected five minutes, two hours, four hours, seven hours, one day, three days, seven days, 14 days, and 28 days after the administration. The collected blood was immediately subjected to separation by centrifugation at 4°C and 15,000 rpm for 15 minutes to obtain the plasma. The separated plasma was stored in a freezer set to -20°C or lower until performing the measurements.

The concentrations of Alprolix and FIX-Fc DL in mouse plasma were determined by ELISA. Specifically, an anti-human FIX antibody-immobilized plate (AssayPro), calibration curve samples containing Alprolix or FIX-FcDL at plasma concentrations of 10.0, 5.00, 2.50, 1.25, 0.625, 0.313, and 0.156 µg/mL, and mouse plasma samples diluted by 100-fold or more were prepared; the samples were aliquoted at 50 µL into each well of the anti-human FIX antibody-immobilized plate, and then this was stirred at room temperature for two hours. Thereafter, biotin anti-human FIX antibody (AssayPro) was allowed to react at room temperature for one hour. Next, the plate after reaction was washed, and further reaction with SP conjugate (AssayPro) was performed at room temperature for 30 minutes. After the reaction, the reaction solution was subjected to a chromogenic reaction using a Chromogen substrate (AssayPro) as the substrate. After stopping the reaction by adding a reaction-stopping solution (AssayPro), the 450-nm absorbance of the reaction solution in each well was measured using a microplate reader. The concentrations of Alprolix and FIX-Fc DL in mouse plasma were calculated from the absorbances of the calibration curve using SOFTmax PRO analysis software (Molecular Devices).

The changes in plasma concentration after intravenous or subcutaneous administration of Alprolix or FIX-Fc DL to mice are shown in Fig. 1. Compared to Alprolix, FIX-Fc DL showed greatly improved exposure, and high plasma concentration was maintained in both intravenous and subcutaneous administrations. PK parameters were calculated from the obtained plasma concentration changes, and are summarized in Table 1.

**Table 1**

| PK parameters in mice for FIX-Fc (Alprolix) and GLA domain-less FIX-Fc (FIX-Fc DL) | | | | |
|---|---|---|---|---|
| Parameter | AUCinf_IV | AUCinf_SC | Bio-availability (BA) | Half-life_SC |
| Units | µg·day/mL | µg·day/mL | % | days |
| Alprolix | 7.39 | 2.41 | 32.6 | 2.02 |
| FIX-Fc DL | 41.2 | 33.4 | 81.1 | 6.99 |

As shown in Table 1, compared to Alprolix, when subcutaneously administered, the half-life of FIX-Fc DL increased by 3.46-fold from 2.02 days to 6.99 days, and the bio-availability improved by 2.49-fold from 32.6% to 81.1%. Therefore, the main cause for the short half-life and low bio-availability of FIX-Fc was found to be attributable to the molecular structure of the GLA domain of FIX.

### [Example 2] Effects of Gla-modification in the GLA domain on pharmacokinetics of FIX-Fc in mice

### 2-1. Preparation of Gla-modification-less FIX-Fc

According to the results from Example 1, the main cause for the short half-life and low bio-availability of FIX-Fc was found to be attributable to the molecular structure of the GLA domain of FIX. Next, examinations were carried out to determine which amino acids in the GLA domain contribute to the short half-life and low bio-availability. Accordingly, we set up a hypothesis that this short half-life is caused by Gla amino acids formed by posttranslational modification of glutamic acid (Glu) to Gla in the GLA domain as Gla-modification. Therefore, a FIX-Fc molecule deficient in Gla-modification (that is, Glu in the GLA domain is maintained as Glu) (FIX-Fc Gla-Modification Less; FIX-Fc ML) was prepared.

Expression of FIX-Fc ML was carried out as follows. Expression vectors encoding FIX-hinge-CH2-CH3 (SEQ ID NO: 4) and hinge-CH2-CH3 (SEQ ID NO: 3) were prepared by a method known to those skilled in the art, and gene transfection was carried out using a transient expression system of Expi 293 (Life Technologies) together with Furin/VKOR. On the second day after gene transfection, vitamin K1 (Sigma-Aldrich) was added together with an enhancer (Life technologies), and the culture supernatant was collected on the fifth day. From the obtained culture supernatant, FIX-Fc ML was purified by a method known to those skilled in the art using HiTrap MabSelect Sure 5 mL (GE Healthcare) and a HiLoad Superdex200 PG 26/60 (GE Healthcare) column, and the concentration was calculated using the PACE method.

The number of Gla in the purified FIX-Fc ML was evaluated according to a known method (Method Mol Biol. 2016 446:85-94). More specifically, FIX-Fc was subjected to alkaline hydrolysis under reduced pressure and nitrogen gas encapsulation using KOH at 110°C for 20 hours, and then this was neutralized with perchloric acid. After neutralization, the hydrolysis product was reacted with o-phthalaldehyde in the presence of ethanethiol for fluorescence labeling. After labeling, analysis by reverse-phase HPLC using a Zorbax Eclipse AAA column was performed to determine the concentration of amino acids in the hydrolysis product. As a result of calculating the number of Gla in FIX-Fc molecules based on the concentration of aspartic acid, the number of Gla in FIX-Fc ML was determined to be 0.6. The number of Gla in Alporlix molecules determined at the same time was 12.4, and this value was close to the reported numerical value of 11.2 ± 0.5 (Blood. 2010 Mar 11;115(10):2057-64).

### 2-2. Pharmacokinetic evaluation of Alprolix and FIX-Fc ML in mice

PK tests using mice were performed by the following method. Alprolix or FIX-Fc ML was administered through the tail vein or under the dorsal skin of mice (C57BL/6J mouse, Charles River Japan) in a single dose at 2 mg/kg. Blood was collected five minutes, two hours, four hours, seven hours, one day, three days, seven days, 14 days, and 28 days after the administration. The collected blood was immediately subjected to separation by centrifugation at 4°C and 15,000 rpm for 15 minutes to obtain the plasma. The separated plasma was stored in a freezer set to -20°C or lower until performing the measurements.

The concentrations of Alprolix and FIX-Fc ML in mouse plasma were determined by ELISA. Specifically, an anti-human FIX antibody-immobilized plate (AssayPro), calibration curve samples containing Alprolix or FIX-Fc ML at plasma concentrations of 10.0, 5.00, 2.50, 1.25, 0.625, 0.313, and 0.156 µg/mL, and mouse plasma samples diluted 100-fold or more were prepared; the samples were aliquoted at 50 µL into each well of the anti-human FIX antibody-immobilized plate, and then this was stirred at room temperature for two hours. Thereafter, biotin anti-human FIX antibody (AssayPro) was allowed to react at room temperature for one hour. The reacted plate was washed, and further reaction with SP conjugate (AssayPro; Streptavidin-Peroxidase Conjugate) was performed at room temperature for 30 minutes. The reaction solution after the reaction was removed, the plate was washed, and a chromogenic reaction was carried out using a Chromogen substrate (AssayPro) as the substrate. After stopping the reaction by adding a reaction-stopping solution (AssayPro), the 450-nm absorbance of the reaction solution in each well was measured using a microplate reader. The concentrations of Alprolix and FIX-Fc ML in mouse plasma were calculated from the absorbances of the calibration curve using SOFTmax PRO analysis software (Molecular Devices).

The changes in plasma concentration after intravenous or subcutaneous administration of Alprolix or FIX-Fc ML to mice are shown in Fig. 2. Compared to Alprolix, FIX-Fc ML showed greatly increased exposure, and high plasma concentration was maintained in both intravenous and subcutaneous administrations. Comparison of Figs. 1 and 2 shows that the plasma concentration changes for FIX-Fc DL and FIX-Fc ML are nearly equivalent. PK parameters were calculated from the obtained plasma concentration changes, and are summarized in Table 2.

**Table 2**

| PK parameters in mice for FIX-Fc (Alprolix) and Gla-modification-less FIX-Fc (FIX-Fc ML) | | | | |
|---|---|---|---|---|
| Parameter | AUCinf_IV | AUCinf_SC | Bio-availability (BA) | Half-life_SC |
| Units | µg·day/mL | µg·day/mL | % | days |
| Alprolix | 7.39 | 2.41 | 32.6 | 2.02 |
| FIX-Fc ML | 77.9 | 66.6 | 85.5 | 7.00 |

As shown in Table 2, compared to Alprolix, when subcutaneously administered, the half-life of FIX-Fc ML increased by 3.47-fold from 2.02 days to 7.00 days, and the bio-availability improved by 2.62-fold from 32.6% to 85.5%. Therefore, the main cause for the short half-life and low bio-availability of FIX-Fc was found to be attributable to the Gla-modification (that is, generation of Gla amino acids by posttranslational modification of glutamic acid) of FIX.

### [Example 3] Pharmacokinetics of FIX-Fc and Gla-modification-less FIX-Fc in cynomolgus monkeys

PK tests using cynomolgus monkeys were performed by the following method. Alprolix or FIX-Fc ML was administered intravenously or subcutaneously to cynomolgus monkeys (from Cambodia) in a single dose at 1 mg/kg. Blood was collected ten minutes, 30 minutes, 2 hours, 7 hours, 1 day, 2 days, 4 days, 7 days, 14 days, 28 days, and 56 days after the administration. The collected blood was immediately subjected to separation by centrifugation at 4°C and 13,000 rpm for 10 minutes to obtain the plasma. The separated plasma was stored in a freezer set to -20°C or lower until performing the measurements.

The concentrations of Alprolix and FIX-Fc ML in cynomolgus monkey plasma were determined by ELISA. Specifically, Anti-Human IgG (Southern Biotech) was dispensed onto Nunc-Immuno Plates, MaxiSoup (Nalge nunc International) and allowed to stand overnight at 5°C, and then this was blocked for one hour using a PBS-Tween solution containing 1% BSA (w/v) and 1 mg/mL Goat IgG, to prepare Anti-Human IgG-immobilized plates. Calibration curve samples containing Alprolix or FIX-Fc ML at plasma concentrations of 3.00, 1.50, 0.750, 0.375, 0.188, 0.0938, and 0.0469 µg/mL, and cynomolgus monkey plasma samples diluted 100-fold or more were prepared; they were aliquoted at 100 µL into each well of the anti-human IgG-immobilized plate, and then this was stirred at room temperature for one hour. Thereafter, reaction with biotin anti-human FIX antibody (prepared in-house) was performed at room temperature for one hour, and then, further reaction with Streptavidin-PolyHRP80 (Stereospecific Detection Technologies) was performed at room temperature for one hour. The reaction solution was removed, the plate was washed, and a chromogenic reaction was carried out using ABTS (Roche Diagnostics) as the substrate. The 405-nm absorbance of the reaction solution in each well was measured on a microplate reader using the 490-nm absorbance as reference. The concentrations of Alprolix and FIX-Fc ML in cynomolgus monkey plasma were calculated from the absorbances of the calibration curve using SOFTmax PRO analysis software (Molecular Devices).

The changes in plasma concentration after intravenous or subcutaneous administration of Alprolix or FIX-Fc ML to cynomolgus monkeys are shown in Fig. 3. Compared to Alprolix, FIX-Fc ML showed greatly increased exposure, and high plasma concentration was maintained in both intravenous and subcutaneous administrations. PK parameters were calculated from the obtained plasma concentration changes, and are summarized in Table 3.

**Table 3**

| PK parameters in cynomolgus monkeys for FIX-Fc (Alprolix) and Gla-modification-less FIX-Fc (FIX-Fc ML). | | | | |
|---|---|---|---|---|
| Parameter | AUCinf_IV | AUCinf_SC | Bio-availability (BA) | Half-life_SC |
| Units | µg·day/mL | µg·day/mL | % | days |
| Alprolix | 13.1 | 3.90 | 29.7 | 4.35 |
| FIX-Fc ML | 65.5 | 37.3 | 56.9 | 7.50 |

As shown in Table 3, compared to Alprolix, when subcutaneously administered, the half-life of FIX-Fc ML increased by 1.72-fold from 4.35 days to 7.50 days, and the bio-availability improved by 1.92-fold from 29.7% to 56.9%. Therefore, the main cause for the short half-life and low bio-availability of FIX-Fc was found to be attributable to the Gla-modification (that is, generation of Gla amino acids by posttranslational modification of glutamic acid) of FIX.

### [Example 4] Effects of administering antibodies that recognize the GLA domain of FIX, on pharmacokinetics of FIX

### 4-1. Preparation of antibodies that recognize the GLA domain of FIX

Expression vectors encoding the heavy chain (SEQ ID NO: 5) and light chain (SEQ ID NO: 6) of antibody A obtained by immunizing an animal using human FIXa, and encoding the heavy chain (SEQ ID NO: 7) and light chain (SEQ ID NO: 8) of antibody B for which its gene was completely synthesized by referring to SB249417 of WO2001087339, were produced by a method known to those skilled in the art, and the antibodies were prepared by a method known to those skilled in the art using a transient expression system of FS293 (Life Technologies). From the obtained culture supernatants, antibodies A and B were purified by a method known to those skilled in the art, and their concentrations were calculated using the PACE method. Antibody A recognizes the EGF domain, whereas antibody B recognizes the GLA domain.

### 4-2. Pharmacokinetic test on simultaneous administration of an anti-FIX antibody and FIX

PK tests using mice were performed by the following method. Through the tail vein or under the dorsal skin of mice (C57BL/6J mouse, Charles River Japan), FIX (Christmassin M I.V.) was administered at a dose of 0.6 mg/kg by itself or simultaneously with 8 mg/kg of an anti-FIX antibody A or anti-FIX antibody B, in a single dose. Blood was collected five minutes, two hours, four hours, seven hours, one day, two days, seven days, 14 days, 21 days, and 28 days after the administration. The collected blood was immediately subjected to separation by centrifugation at 4°C and 15,000 rpm for 15 minutes to obtain the plasma. The separated plasma was stored in a freezer set to -20°C or lower until performing the measurements.

The concentration of FIX in mouse plasma was determined by ELISA. Specifically, an anti-human FIX antibody-immobilized plate (AssayPro), calibration curve samples containing FIX at plasma concentrations of 10.0, 5.00, 2.50, 1.25, 0.625, 0.313, and 0.156 µg/mL, and mouse plasma assay samples diluted by 100-fold or more were prepared; the samples were aliquoted at 50 µL into each well of the anti-human FIX antibody-immobilized plate, and then this was stirred at room temperature for two hours. Thereafter, reaction with biotin anti-human FIX antibody (AssayPro) was performed at room temperature for one hour. The reacted plate was washed, and then, further reaction with SP conjugate (AssayPro) was performed at room temperature for 30 minutes. The reaction solution was removed, and the plate was washed, and then a chromogenic reaction was performed using a Chromogen substrate (AssayPro) as the substrate. After stopping the reaction by adding a reaction-stopping solution (AssayPro), the 450-nm absorbance of the reaction solution in each well was measured using a microplate reader. The concentrations of FIX in mouse plasma were calculated from the absorbances of the calibration curve using SOFTmax PRO analysis software (Molecular Devices).

The concentration of FIX in mouse plasma in the presence of anti-FIX antibody A or anti-FIX antibody B was determined by ELISA. Specifically, an anti-human FIX antibody-immobilized plate (AssayPro), calibration curve samples containing FIX at plasma concentrations of 10.0, 5.00, 2.50, 1.25, 0.625, 0.313, and 0.156 µg/mL, and mouse plasma samples diluted by 100-fold or more were prepared using an assay buffer containing 2 µg/mL of an anti-FIX antibody A or anti-FIX antibody B; the samples were aliquoted at 50 µL into each well of the anti-human FIX antibody-immobilized plate (AssayPro), and then this was stirred at room temperature for two hours. Thereafter, reaction with biotin anti-human FIX antibody (AssayPro) was performed at room temperature for one hour. The reacted plate was washed, and further reaction with SP conjugate (AssayPro) was performed at room temperature for 30 minutes. The reaction solution was removed, and the plate was washed, and then a chromogenic reaction was performed using a Chromogen substrate (AssayPro) as the substrate. After stopping the reaction by adding a reaction-stopping solution (AssayPro), the 450-nm absorbance of the reaction solution in each well was measured using a microplate reader. The concentrations of FIX in mouse plasma were calculated from the absorbances of the calibration curve using SOFTmax PRO analysis software (Molecular Devices).

The changes in plasma concentration of FIX after intravenous or subcutaneous administration of FIX alone or simultaneously with an anti-FIX antibody A or anti-FIX antibody B to mice are shown in Fig. 4. Compared to FIX alone, simultaneously administering FIX with an anti-FIX antibody A or anti-FIX antibody B greatly increased the exposure of FIX. The effect on increased exposure of FIX was stronger with anti-FIX antibody B than with anti-FIX antibody A, and high plasma concentration was maintained in both intravenous and subcutaneous administrations. PK parameters were calculated from the obtained plasma concentration changes, and the results are summarized in Table 4.

**Table 4**

| PK parameters for FIX in mice concerning single administration of FIX and simultaneous administration of FIX with antibody A or antibody B. | | | | | |
|---|---|---|---|---|---|
| | | | Bio-availability | | |
| Parameter | AUCinf_IV | AUCinf_SC | (BA) | Half-life_IV | Cmax_SC |
| Units | µg·day/mL | µg·day/mL | % | days | µg/mL |
| FIX | 0.901 | NC | NC | 0.242 | ND |
| FIX + anti-FIX antibody A | 2.53 | NC | NC | 1.01 | 0.217 |
| FIX + anti-FIX antibody B | 18.7 | 11.8 | 63.1 | 3.13 | 2.16 |

As shown in Table 4, by using anti-FIX antibody B to inhibit the function of Gla-modification which causes worsening of pharmacokinetics, plasma half-life of FIX increased by 3.10-fold from 1.01 days yielded by anti-FIX antibody A to 3.13 days. Cmax of FIX was 0.217 µg/mL when subcutaneously administered simultaneously with anti-FIX antibody A, whereas the Cmax became 2.16 µg/mL when subcutaneously administered simultaneously with anti-FIX antibody B, and resulted in a 9.95-fold increase. Furthermore, while the BA of FIX could not be calculated when subcutaneously administrated simultaneously with anti-FIX antibody A, the BA when subcutaneously administered simultaneously with anti-FIX antibody B was 63.1% and was satisfactory.

Accordingly, neutralization of Gla-modification or the GLA domain by an antibody was confirmed to be able to greatly improve the short half-life and low bio-availability of FIX-Fc.

### [Example 5] Preparation of FIX-Fc with controlled number of Gla-modifications through expression conditions, and evaluation of the pharmacokinetics of such FIX-Fc

### 5-1. Preparation of FIX-Fc with controlled number of Gla-modifications

Expression vectors in which the glutamic acid (E)-encoding nucleotide sequence in the GLA domain of FIX-Fc have been changed one by one in the order from the N terminus to a nucleotide sequence encoding glutamine (Q) or aspartic acid (D) were produced. When FIX-Fc molecules are expressed using these vectors, FIX-Fc molecules in which one position among the twelve positions are not Gla-modified are expected to be expressed. Using these expression vectors, FIX-Fc molecules with controlled number of Gla-modifications (FIX-Fc Modification controlled; FIX-Fc MC) were prepared.

FIX-Fc MC was expressed as follows. Expression vectors encoding FIX-hinge-CH2-CH3 (SEQ ID NO: 4) and hinge-CH2-CH3 (SEQ ID NO: 5) were prepared by a method known to those skilled in the art, and gene transfection together with Furin/VKOR was carried out using a transient expression system of Expi 293 (Life Technologies). On the second day after gene transfection, vitamin K1 (Sigma-Aldrich) was added together with an enhancer (Life technologies), and the culture supernatant was collected on the fifth day. From the obtained culture supernatant, FIX-Fc MC was purified by a method known to those skilled in the art using HiTrap MabSelect Sure 5 mL (GE Healthcare) and a HiLoad Superdex200 PG 26/60 (GE Healthcare) column, and the concentration was calculated using the PACE method.

The number of Gla in the purified FIX-Fc MC is evaluated by the method described in Example 2 (Method Mol Biol. 2016 446:85-94; and Blood. 2010 Mar 11;115(10):2057-64).

### 5-2. Pharmacokinetic test of simultaneous administration of an anti-FIX antibody and FIX-Fc MC

PK tests using mice are performed by the following method. Through the tail vein or under the dorsal skin of mice (C57BL/6J mouse, Charles River Japan), FIX-Fc MC is administered by itself or simultaneously with anti-FIX antibody A or anti-FIX antibody B, in a single dose. After the administration, blood is collected at specified times, and they are subjected to centrifugation to obtain the plasma. The concentration of FIX-Fc MC in mouse plasma is determined by ELISA. PK parameters are calculated from plasma concentration shifts.

### Industrial Applicability

The present invention improved either or preferably both the blood pharmacokinetics and the bio-availability of FIX. Improvement of the blood pharmacokinetics of FIX allows patients needing a FIX administration to benefit from equivalent effects even when the administration is carried out with an administration interval that is longer compared to that for known FIX formulations. Alternatively, improvement of the bio-availability of FIX enables subcutaneous administration to patients needing a FIX administration, and the administration does not need to depend on intravenous administration. Unlike intravenous administration which has limitations on the site of administration to sites that allow access to the veins from the body surface, subcutaneous administration is a method that allows administration to a wide range of areas. Therefore, therapeutic burden can be lowered particularly for patients with FIX deficiency disease, who need long-term administration.

## Claims

1. A blood coagulation factor IX having either or both of an improved plasma half-life and an improved bio-availability, which comprises a modified GLA domain.

2. The blood coagulation factor IX of claim 1, wherein the modification is one or more modifications selected from the group consisting of:
(i) non-covalent bonding of a GLA-domain-recognizing antibody or an antibody fragment thereof to the GLA domain;
(ii) reduced number of Gla residues in the GLA domain, in comparison to that of a native blood coagulation factor IX;
(iii) either or both of deletion of one or more glutamic acid residues in the GLA domain and substitution of one or more glutamic acid residues in the GLA domain with another amino acid; and
(iv) deletion of a part or all of the GLA domain.

3. The blood coagulation factor IX of claim 2, wherein the antibody fragment is Fab, F(ab')2, or scFv.

4. The blood coagulation factor IX of any one of claims 1 to 3, wherein the blood coagulation factor IX is a fusion protein formed with an FcRn-binding protein, or a fusion protein formed with an FcRn-binding protein and a GLA-domain-recognizing antibody or an antibody fragment thereof.

5. A method for improving either or both of the plasma half-life and bio-availability of a blood coagulation factor IX, which comprises the step of modifying a GLA domain.

6. The method of claim 5, wherein the modifying step is one or more steps selected from the group consisting of:
(i) non-covalently binding a GLA-domain-recognizing antibody or an antibody fragment thereof to the GLA domain;
(ii) reducing the number of Gla residues in the GLA domain, in comparison to that of a native blood coagulation factor IX;
(iii) carrying out either or both of deletion of one or more glutamic acid residues in the GLA domain and substitution of one or more glutamic acid residues in the GLA domain with another amino acid; and
(iv) deleting a part or all of the GLA domain.

7. A method for controlling either or both of the plasma half-life and bio-availability of a blood coagulation factor IX, which comprises the step of modifying a GLA domain.

8. The method of claim 7, wherein the modifying step is one or more steps selected from the group consisting of:
(i) non-covalently binding a GLA-domain-recognizing antibody or an antibody fragment thereof to the GLA domain;
(ii) reducing the number of Gla residues in the GLA domain, in comparison to that of a native blood coagulation factor IX;
(iii) carrying out either or both of deletion of one or more glutamic acid residues in the GLA domain and substitution of one or more glutamic acid residues in the GLA domain with another amino acid; and
(iv) deleting a part or all of the GLA domain.

9. A method for producing a blood coagulation factor IX with either or both of an improved plasma half-life and an improved bio-availability of the blood coagulation factor IX, which comprises the step of modifying a GLA domain.

10. The method of claim 9, wherein the modifying step is one or more steps selected from the group consisting of:
(i) non-covalently binding a GLA-domain-recognizing antibody or an antibody fragment thereof to the GLA domain;
(ii) reducing the number of Gla residues in the GLA domain, in comparison to that of a native blood coagulation factor IX;
(iii) carrying out either or both of deletion of one or more glutamic acid residues in the GLA domain and substitution of one or more glutamic acid residues in the GLA domain with another amino acid; and
(iv) deleting a part or all of the GLA domain.

11. The method of claim 9 or 10, which further comprises the step of isolating a blood coagulation factor IX with a modified GLA domain.

12. A complex formed between a blood coagulation factor IX and a GLA-domain-recognizing antibody or an antibody fragment thereof.

13. A pharmaceutical composition which comprises the blood coagulation factor IX of any one of claims 1 to 4, a blood coagulation factor IX produced by the method of any one of claims 9 to 11, or the complex of claim 12 as an active ingredient.

14. The pharmaceutical composition of claim 13, which is used for either or both of prevention and treatment of a FIX deficiency disease.

15. The pharmaceutical composition of claim 14, wherein the FIX deficiency disease is hemophilia B.


SEQUENCE LISTING

<110>  CHUGAI SEIYAKU KABUSHIKI KAISHA

<120>  BLOOD COAGULATION FACTOR IX WITH ENHANCED PHARMACOKINETICS

<130>  C1-A1617P

<150>  JP 2017-088670
<151>  2017-04-27

<160>  8

<170>  PatentIn version 3.5

<210>  1
<211>  461
<212>  PRT
<213>  Homo sapiens

<400>  1

<210>  2
<211>  594
<212>  PRT
<213>  Artificial sequence

<220>
<223>  an artificially synthesized sequence

<400>  2

Asp Gly Asp Gln Cys Glu Ser Asn Pro Cys Leu Asn Gly Gly Ser Cys
1               5                   10                  15

Lys Asp Asp Ile Asn Ser Tyr Glu Cys Trp Cys Pro Phe Gly Phe Glu
            20                  25                  30

Gly Lys Asn Cys Glu Leu Asp Val Thr Cys Asn Ile Lys Asn Gly Arg
        35                  40                  45

Cys Glu Gln Phe Cys Lys Asn Ser Ala Asp Asn Lys Val Val Cys Ser
    50                  55                  60

Cys Thr Glu Gly Tyr Arg Leu Ala Glu Asn Gln Lys Ser Cys Glu Pro
65                  70                  75                  80

Ala Val Pro Phe Pro Cys Gly Arg Val Ser Val Ser Gln Thr Ser Lys
                85                  90                  95

Leu Thr Arg Ala Glu Thr Val Phe Pro Asp Val Asp Tyr Val Asn Ser
            100                 105                 110

Thr Glu Ala Glu Thr Ile Leu Asp Asn Ile Thr Gln Ser Thr Gln Ser
        115                 120                 125

Phe Asn Asp Phe Thr Arg Val Val Gly Gly Glu Asp Ala Lys Pro Gly
    130                 135                 140

Gln Phe Pro Trp Gln Val Val Leu Asn Gly Lys Val Asp Ala Phe Cys
145                 150                 155                 160

Gly Gly Ser Ile Val Asn Glu Lys Trp Ile Val Thr Ala Ala His Cys

                165                 170                 175

Val Glu Thr Gly Val Lys Ile Thr Val Val Ala Gly Glu His Asn Ile
            180                 185                 190

Glu Glu Thr Glu His Thr Glu Gln Lys Arg Asn Val Ile Arg Ile Ile
        195                 200                 205

Pro His His Asn Tyr Asn Ala Ala Ile Asn Lys Tyr Asn His Asp Ile
    210                 215                 220

Ala Leu Leu Glu Leu Asp Glu Pro Leu Val Leu Asn Ser Tyr Val Thr
225                 230                 235                 240

Pro Ile Cys Ile Ala Asp Lys Glu Tyr Thr Asn Ile Phe Leu Lys Phe
                245                 250                 255

Gly Ser Gly Tyr Val Ser Gly Trp Gly Arg Val Phe His Lys Gly Arg
            260                 265                 270

Ser Ala Leu Val Leu Gln Tyr Leu Arg Val Pro Leu Val Asp Arg Ala
        275                 280                 285

Thr Cys Leu Arg Ser Thr Lys Phe Thr Ile Tyr Asn Asn Met Phe Cys
    290                 295                 300

Ala Gly Phe His Glu Gly Gly Arg Asp Ser Cys Gln Gly Asp Ser Gly
305                 310                 315                 320

Gly Pro His Val Thr Glu Val Glu Gly Thr Ser Phe Leu Thr Gly Ile
                325                 330                 335

Ile Ser Trp Gly Glu Glu Cys Ala Met Lys Gly Lys Tyr Gly Ile Tyr
            340                 345                 350

Thr Lys Val Ser Arg Tyr Val Asn Trp Ile Lys Glu Lys Thr Lys Leu
        355                 360                 365

Thr Asp Lys Thr His Thr Cys Pro Pro Cys Pro Ala Pro Glu Leu Arg
    370                 375                 380

Arg Gly Pro Ser Val Phe Leu Phe Pro Pro Lys Pro Lys Asp Thr Leu
385                 390                 395                 400

Met Ile Ser Arg Thr Pro Glu Val Thr Cys Val Val Val Asp Val Ser
                405                 410                 415

His Glu Asp Pro Glu Val Lys Phe Asn Trp Tyr Val Asp Gly Val Glu
            420                 425                 430

Val His Asn Ala Lys Thr Lys Pro Arg Glu Glu Gln Tyr Asn Ser Thr
        435                 440                 445

Tyr Arg Val Val Ser Val Leu Thr Val Leu His Gln Asp Trp Leu Asn
    450                 455                 460

Gly Lys Glu Tyr Lys Cys Lys Val Ser Asn Lys Gly Leu Pro Ser Ser
465                 470                 475                 480

Ile Glu Lys Thr Ile Ser Lys Ala Lys Gly Gln Pro Arg Glu Pro Gln
                485                 490                 495

Val Tyr Thr Leu Pro Pro Ser Arg Cys Glu Met Thr Lys Asn Gln Val
            500                 505                 510

Ser Leu Ser Cys Ala Val Lys Gly Phe Tyr Pro Ser Asp Ile Ala Val
        515                 520                 525

Glu Trp Glu Ser Asn Gly Gln Pro Glu Asn Asn Tyr Lys Thr Thr Pro
    530                 535                 540

Pro Val Leu Asp Ser Asp Gly Ser Phe Phe Leu Val Ser Lys Leu Thr
545                 550                 555                 560

Val Asp Lys Ser Arg Trp Gln Glu Gly Asn Val Phe Ser Cys Ser Val
                565                 570                 575

Met His Glu Ala Leu His Asn Arg Tyr Thr Gln Lys Ser Leu Ser Leu
            580                 585                 590

Ser Pro

<210>  3
<211>  225
<212>  PRT
<213>  Artificial sequence

<220>
<223>  an artificially synthesized sequence

<400>  3

Asp Lys Thr His Thr Cys Pro Pro Cys Pro Ala Pro Glu Leu Arg Arg
1               5                   10                  15

Gly Pro Ser Val Phe Leu Phe Pro Pro Lys Pro Lys Asp Thr Leu Met

20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Gly Leu Pro Ser Ser Ile
            100                 105                 110

Glu Lys Thr Ile Ser Lys Ala Lys Gly Gln Pro Arg Glu Pro Gln Val
        115                 120                 125

Cys Thr Leu Pro Pro Ser Arg Glu Glu Met Thr Lys Asn Gln Val Ser
    130                 135                 140

Leu Trp Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Glu Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro
225

<210>  4
<211>  640
<212>  PRT
<213>  Artificial sequence

<220>
<223>  an artificially synthesized sequence

<400>  4

Tyr Asn Ser Gly Lys Leu Glu Glu Phe Val Gln Gly Asn Leu Glu Arg
1               5                   10                  15

Glu Cys Met Glu Glu Lys Cys Ser Phe Glu Glu Ala Arg Glu Val Phe
            20                  25                  30

Glu Asn Thr Glu Arg Thr Thr Glu Phe Trp Lys Gln Tyr Val Asp Gly
        35                  40                  45

Asp Gln Cys Glu Ser Asn Pro Cys Leu Asn Gly Gly Ser Cys Lys Asp
    50                  55                  60

Asp Ile Asn Ser Tyr Glu Cys Trp Cys Pro Phe Gly Phe Glu Gly Lys
65                  70                  75                  80

Asn Cys Glu Leu Asp Val Thr Cys Asn Ile Lys Asn Gly Arg Cys Glu
                85                  90                  95

Gln Phe Cys Lys Asn Ser Ala Asp Asn Lys Val Val Cys Ser Cys Thr
            100                 105                 110

Glu Gly Tyr Arg Leu Ala Glu Asn Gln Lys Ser Cys Glu Pro Ala Val
        115                 120                 125

Pro Phe Pro Cys Gly Arg Val Ser Val Ser Gln Thr Ser Lys Leu Thr
    130                 135                 140

Arg Ala Glu Thr Val Phe Pro Asp Val Asp Tyr Val Asn Ser Thr Glu
145                 150                 155                 160

Ala Glu Thr Ile Leu Asp Asn Ile Thr Gln Ser Thr Gln Ser Phe Asn
                165                 170                 175

Asp Phe Thr Arg Val Val Gly Gly Glu Asp Ala Lys Pro Gly Gln Phe
            180                 185                 190

Pro Trp Gln Val Val Leu Asn Gly Lys Val Asp Ala Phe Cys Gly Gly
        195                 200                 205

Ser Ile Val Asn Glu Lys Trp Ile Val Thr Ala Ala His Cys Val Glu
    210                 215                 220

Thr Gly Val Lys Ile Thr Val Val Ala Gly Glu His Asn Ile Glu Glu
225                 230                 235                 240

Thr Glu His Thr Glu Gln Lys Arg Asn Val Ile Arg Ile Ile Pro His

245                 250                 255

His Asn Tyr Asn Ala Ala Ile Asn Lys Tyr Asn His Asp Ile Ala Leu
        260                 265                 270

Leu Glu Leu Asp Glu Pro Leu Val Leu Asn Ser Tyr Val Thr Pro Ile
        275                 280                 285

Cys Ile Ala Asp Lys Glu Tyr Thr Asn Ile Phe Leu Lys Phe Gly Ser
    290                 295                 300

Gly Tyr Val Ser Gly Trp Gly Arg Val Phe His Lys Gly Arg Ser Ala
305                 310                 315                 320

Leu Val Leu Gln Tyr Leu Arg Val Pro Leu Val Asp Arg Ala Thr Cys
                325                 330                 335

Leu Arg Ser Thr Lys Phe Thr Ile Tyr Asn Asn Met Phe Cys Ala Gly
            340                 345                 350

Phe His Glu Gly Gly Arg Asp Ser Cys Gln Gly Asp Ser Gly Gly Pro
        355                 360                 365

His Val Thr Glu Val Glu Gly Thr Ser Phe Leu Thr Gly Ile Ile Ser
    370                 375                 380

Trp Gly Glu Glu Cys Ala Met Lys Gly Lys Tyr Gly Ile Tyr Thr Lys
385                 390                 395                 400

Val Ser Arg Tyr Val Asn Trp Ile Lys Glu Lys Thr Lys Leu Thr Asp
                405                 410                 415

Lys Thr His Thr Cys Pro Pro Cys Pro Ala Pro Glu Leu Arg Arg Gly
            420                 425                 430

Pro Ser Val Phe Leu Phe Pro Pro Lys Pro Lys Asp Thr Leu Met Ile
        435                 440                 445

Ser Arg Thr Pro Glu Val Thr Cys Val Val Val Asp Val Ser His Glu
    450                 455                 460

Asp Pro Glu Val Lys Phe Asn Trp Tyr Val Asp Gly Val Glu Val His
465                 470                 475                 480

Asn Ala Lys Thr Lys Pro Arg Glu Glu Gln Tyr Asn Ser Thr Tyr Arg
                485                 490                 495

Val Val Ser Val Leu Thr Val Leu His Gln Asp Trp Leu Asn Gly Lys
            500                 505                 510

Glu Tyr Lys Cys Lys Val Ser Asn Lys Gly Leu Pro Ser Ser Ile Glu
        515                 520                 525

Lys Thr Ile Ser Lys Ala Lys Gly Gln Pro Arg Glu Pro Gln Val Tyr
    530                 535                 540

Thr Leu Pro Pro Ser Arg Cys Glu Met Thr Lys Asn Gln Val Ser Leu
545                 550                 555                 560

Ser Cys Ala Val Lys Gly Phe Tyr Pro Ser Asp Ile Ala Val Glu Trp
                565                 570                 575

Glu Ser Asn Gly Gln Pro Glu Asn Asn Tyr Lys Thr Thr Pro Pro Val
            580                 585                 590

Leu Asp Ser Asp Gly Ser Phe Phe Leu Val Ser Lys Leu Thr Val Asp
        595                 600                 605

Lys Ser Arg Trp Gln Glu Gly Asn Val Phe Ser Cys Ser Val Met His
    610                 615                 620

Glu Ala Leu His Asn Arg Tyr Thr Gln Lys Ser Leu Ser Leu Ser Pro
625                 630                 635                 640

<210>  5
<211>  446
<212>  PRT
<213>  Artificial sequence

<220>
<223>  an artificially synthesized sequence

<400>  5

Gln Val Gln Leu Gln Gln Ser Gly Ala Glu Leu Ala Lys Pro Gly Ala
1               5                   10                  15

Ser Val Lys Leu Ser Cys Lys Ala Ser Gly Tyr Thr Phe Thr Ser Ser
            20                  25                  30

Trp Met His Trp Ile Lys Gln Arg Pro Gly Gln Gly Leu Glu Trp Leu
        35                  40                  45

Gly Tyr Ile Asn Pro Ser Ser Gly Tyr Thr Lys Tyr Asn Arg Lys Phe
    50                  55                  60

Arg Asp Lys Ala Thr Leu Thr Ala Asp Lys Ser Ser Ser Thr Ala Tyr

65                  70                  75                  80

Met Gln Leu Thr Ser Leu Thr Tyr Glu Asp Ser Ala Val Tyr Tyr Cys
                85                  90                  95

Ala Arg Gly Gly Asn Gly Tyr Tyr Phe Asp Tyr Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Arg Val Glu Pro Lys Ser Cys Asp Lys Thr
    210                 215                 220

His Thr Cys Pro Pro Cys Pro Ala Pro Glu Leu Arg Arg Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Glu Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Glu Gly Asn Val Phe Ser Cys Ser Val Met His Glu Ala
            420                 425                 430

Leu His Asn His Tyr Thr Gln Lys Ser Leu Ser Leu Ser Pro
        435                 440                 445

<210> 6
<211> 213
<212> PRT
<213> Artificial sequence

<220>
<223> an artificially synthesized sequence

<400> 6

Asp Ile Val Met Thr Gln Ser His Lys Phe Met Ser Thr Ser Val Gly
1               5                   10                  15

Asp Arg Val Ser Ile Thr Cys Lys Ala Ser Gln Asp Val Gly Thr Ala
            20                  25                  30

Val Ala Trp Tyr Gln Gln Lys Pro Gly Gln Ser Pro Lys Leu Leu Ile
        35                  40                  45

Tyr Trp Ala Ser Thr Arg His Thr Gly Val Pro Asp Arg Phe Thr Gly
    50                  55                  60

Ser Arg Tyr Gly Thr Asp Phe Thr Leu Thr Ile Ser Asn Val Gln Ser
65                  70                  75                  80

Glu Asp Leu Ala Asp Tyr Leu Cys Gln Gln Tyr Ser Asn Tyr Ile Thr

85                  90                  95

Phe Gly Gly Gly Thr Lys Leu Glu Leu Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  7
<211>  449
<212>  PRT
<213>  Artificial sequence

<220>
<223>  an artificially synthesized sequence

<400>  7

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Asn Tyr
            20                  25                  30

Gly Met Asn Trp Val Arg Gln Ala Pro Gly Gln Gly Leu Lys Trp Met
        35                  40                  45

Gly Trp Ile Asn Thr Arg Asn Gly Lys Ser Thr Tyr Val Asp Asp Phe
    50                  55                  60

Lys Gly Arg Phe Val Phe Ser Leu Asp Ser Ser Val Ser Thr Ala Tyr
65                  70                  75                  80

Leu Gln Ile Ser Ser Leu Lys Ala Asp Asp Thr Ala Val Tyr Tyr Cys
                85                  90                  95

Thr Arg Glu Gly Asn Met Asp Gly Tyr Phe Pro Phe Thr Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Arg Val Glu Pro Lys Ser Cys
    210                 215                 220

Asp Lys Thr His Thr Cys Pro Pro Cys Pro Ala Pro Glu Leu Arg Arg
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Gly Leu Pro Ser Ser Ile

325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Glu Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro

<210>  8
<211>  214
<212>  PRT
<213>  Artificial sequence

<220>
<223>  an artificially synthesized sequence

<400>  8

Gln Ile Val Leu Thr Gln Ser Pro Ser Ser Leu Ser Ala Ser Val Gly
1               5                   10                  15

Asp Arg Val Thr Ile Thr Cys Arg Ala Ser Ser Ser Val Asn Tyr Met
            20                  25                  30

His Trp Tyr Gln Gln Lys Pro Gly Lys Ala Pro Lys Pro Trp Ile Tyr
        35                  40                  45

Ala Thr Ser Asn Leu Ala Ser Gly Val Pro Ser Arg Phe Ser Gly Ser
    50                  55                  60

Gly Ser Gly Thr Asp Tyr Thr Phe Thr Ile Ser Ser Leu Gln Pro Glu
65                  70                  75                  80

Asp Ile Ala Thr Tyr Tyr Cys Gln Gln Trp Ser Ile Asn Pro Arg Thr
                85                  90                  95

Phe Gly Gln Gly Thr Lys Val Glu Ile Lys Arg Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210